# EUROPÄISCHE PATENTSCHRIFT

(11) **EP 4 163 077 B1**
(45) Veröffentlichungstag und Bekanntmachung des Hinweises auf die Patenterteilung: **31.07.2024**
(21) Anmeldenummer: 21201727.1
(22) Anmeldetag: 08.10.2021
(51) Int. Cl.: B29C 35/08, B29C 64/10, B29C 64/264, B33Y 10/00, B33Y 30/00, A61B 18/18, A61N 2/00, A61N 5/02, G01N 24/10, G01R 33/60

(54) **VORRICHTUNG SOWIE VERFAHREN ZUM BEARBEITEN EINER 3D-POLYMERSTRUKTUR**
DEVICE AND METHOD FOR PROCESSING A 3D POLYMER STRUCTURE
DISPOSITIF, AINSI QUE PROCÉDÉ D'USINAGE D'UNE STRUCTURE POLYMÈRE 3D

(43) Veröffentlichungstag der Anmeldung: 12.04.2023
(73) Patentinhaber: upolluX GmbH & Co. KG, 78532 Tuttlingen (DE)
(72) Erfinder: Baumann, Daniel, 78532 Tuttlingen (DE)
(74) Vertreter: Kohler Schmid Möbus Patentanwälte

(56) Entgegenhaltungen:
- EP-A1- 4 094 926
- WO-A1-2005/119286
- US-A1- 2016 227 876
- US-A1- 2021 283 838

## Beschreibung

Die vorliegende Erfindung betrifft eine Vorrichtung sowie ein Verfahren zum Bearbeiten einer dreidimensionalen (= 3D-) Polymerstruktur.

Zur Bearbeitung von 3D-Polymerstrukturen werden in der Praxis unterschiedliche mechanische, thermische oder chemische Bearbeitungsverfahren eingesetzt. So können beispielsweise spanende Verfahren oder auch thermische Verfahren, etwa mit Laserstrahlung, eingesetzt werden.

Ein wesentliches Problem der bekannten Bearbeitungsverfahren besteht in deren limitierter räumlichen Auflösung, dem nur von außen möglichen Werkzeugangriff sowie der mitunter schwierigen Prozessführung.

Es ist deshalb die Aufgabe der Erfindung, eine Vorrichtung und ein Verfahren anzugeben, mit denen eine 3D-Polymerstruktur auf einfache und hochpräzise Weise in vitro d. h. im menschlichen/tierischen Körper, mit einem bislang unerreichten Auflösungsvermögen bearbeitet werden kann.

WO2005/119286 offenbart eine Vorrichtung gemäß dem Oberbegriff des Anspruchs 1.

### Lösung der erfindungsgemäßen Aufgabe

Die die Vorrichtung betreffende Aufgabe wird durch eine Vorrichtung mit den in Anspruch 1 angegebenen Merkmalen und die das Verfahren betreffende Aufgabe wird durch ein Verfahren mit den in Anspruch 5 angegebenen Merkmalen gelöst.

### Vorrichtung

Die erfindungsgemäße Vorrichtung gemäß Anspruch 1 dient dem Bearbeiten einer 3D-Polymerstruktur mit einer im Material der 3D-Polymerstruktur möglichst homogen verteilt angeordneten paramagnetischen (bzw. superparamagnetischen) Substanz.

Die erfindungsgemäße Vorrichtung erlaubt ein hochpräzises Bearbeiten von 3D-Polymerstrukturen mit überlegener räumlicher Auflösung. Dies durch HF-Stimulation der paramagnetischen Substanz in definierten elektromagnetischen Resonanznischen, welche ortsspezifisch, insbesondere auf der Basis hochauflösender Bilddaten, durch gezielte Überlagerung magnetischer Gradientenfelder innerhalb der 3D-Polymerstruktur erzeugt werden. Die 3D-Polymerstruktur kann dabei jedwede Geometrie, Struktur, Oberflächengestaltung und in Abhängigkeit der eingesetzten Materialien auch unterschiedliche Materialeigenschaften aufweisen. Ein- und mehrzeitige Nachbearbeitungsprozeduren der 3D-Polymerstruktur sind möglich. Dadurch lassen sich ein bislang nicht oder nur schwer realisierbarer räumlicher Aufbau und Oberflächenbeschaffenheit der 3D-Polymerstruktur erreichen.

Die Vorrichtung kann nach einer bevorzugten Weiterbildung der Erfindung darüber hinaus auch dem Erzeugen der 3D-Polymerstruktur aus einer Polymervorstufe (= Präpolymer oder Polymervorlage) mit einer im Material der Polymervorstufe (homogen oder im Wesentlichen homogen) verteilt angeordneten paramagnetischen Substanz dienen. Die Polymervorstufe ist im Arbeitsbereich der Vorrichtung anordenbar. Mittels der Gradientenspulen sind magnetische Gradientenfelder B₁, B₂, B₃ in mindestens allen drei Raumrichtungen x, y, z generierbar, um die paramagnetische Substanz zeitgleich bzw. zeitlich sequenziell in definierten Voxeln V der Polymervorstufe dreidimensional ortszucodieren. Die Steuerungseinheit ist bei dieser Ausführungsform der Vorrichtung zusätzlich dazu eingerichtet bzw. programmiert, den HF-Feldgenerator derart anzusteuern, dass die ortskodierte paramagnetische Substanz im dem jeweilig Voxel V mittels einer auf die paramagnetische Substanz abgestimmten Feldfrequenz der HF-Strahlung derart anregbar ist, dass eine thermische Polymerisation der Polymervorstufe in dem definierten Voxel V ermöglicht ist.

Mit dieser Ausführungsform der Vorrichtung kann mithin die 3D-Polymerstruktur einerseits erzeugt als auch nachbearbeitet werden.

Die paramagnetische Substanz ist im Material der Polymervorstufe/des 3D-Polymerstruktur bevorzugt homogen bzw. im Wesentlichen homogen verteilt angeordnet. Dies ist für ein reproduzierbare definierte Ortsauflösung bei der Fertigung der 3D-Polymerstruktur bzw. bei deren Nachbearbeitung vorteilhaft.

Die Vorrichtung gemäß Anspruch 1 umfasst eine Einrichtung zur Bilddatenakquisition bzw. Bildakquisition umfassen. Mit anderen Worten eine Einrichtung zum Gewinnen von Bilddaten. Besonders bevorzugt umfasst die Vorrichtung ein MRT-Gerät. Ein solches MRT-Gerät ermöglicht die Akquisition von Bilddaten aus dem Arbeitsbereich, mithin der 3D-Polymerstruktur bzw. der Polymervorstufe, sowie bedarfsweise auch der jeweiligen Umgebung. MRT-Geräte können funktionell durch eine geeignete Programmierung ihrer Steuerungssoftware zu einer erfindungsgemäßen Vorrichtung erweitert werden. Es versteht sich, dass dies grundsätzlich auch für bereits existierende MRT-Geräten gilt.

Die Vorrichtung kann nach der Erfindung zusätzlich oder alternativ einen Computertomographen (= CT), einen digitalen Volumentomographen (= DVT), ein Sonographiegerät, einen LASER-Scanner und/oder einen Positronen-Emissions-Tomographen (= PET) umfassen, um geeignete Bilddaten aus den vorgenannten Bereichen der 3D-Polymerstruktur bzw. der Polymervorstufe zu akquirieren.

Zur Gewinnung von Bilddaten kann die Vorrichtung zudem eine sogenannte Magnetpartikelbildgebung (=Magnetic Particle Imaging, kurz "MPI") aufweisen bzw. für diese eingerichtet sein. Dadurch kann spezifisch die Verteilung der (para)magnetischen Substanz in einem Volumen des Polymermaterials bzw. der Polymervorstufe bestimmt werden. Anders als bei MRT, wo der Einfluss des jeweilig untersuchten Materials (=Polymermaterial/Polymervorstufe) selbst (Magnetresonanzeigenschaften von Protonen) gemessen wird, ist anhand der durch die MPI-Funktionalität der Vorrichtung erreichbaren Magnetisierung des magnetischen Materials bzw. der paramagnetischen Substanz) Ort und Konzentration desselben bestimmbar.

Die Steuerungseinheit der Vorrichtung weist vorzugsweise einen Betriebsmodus zum Gewinnen und Auswerten von Bilddaten, insbesondere magnetresonanztomografischen Daten, aus dem Arbeitsbereich der Vorrichtung auf. Dadurch können die Bilddaten aus der 3D-Polymerstruktur bzw. der Polymervorstufe sowie bedarfsweise auch der jeweiligen Umgebung vor, während und nach dem Fertigungs- bzw. Bearbeitungsprozess gewonnen werden. Dadurch erlaubt die Vorrichtung, eine bildgebende Diagnostik bei der Bearbeitung bzw. Fertigung der 3D-Polymerstruktur einzubinden bzw. die Logik elektromagnetischer und biomimetischer Induktionsprinzipien zu kombinieren.

Umfasst die Vorrichtung ein MRT bzw. MPI-Funktionalität, so können zudem Thermometriedaten im Arbeitsbereich, d. h. aus der 3D-Polymerstruktur bzw. der Polymervorstufe, sowie bedarfsweise der jeweiligen Umgebung (hier insbesondere Gewebestrukturen in vivo) gewonnen werden. Darüber hinaus werden MRTgestützte Elastizitätsmessungen (= Magnetresonanz-Elastographie) bzw. MPIgestützte Viskositätsmessungen in den vorgenannten Bereichen ermöglicht. Die vorstehend genannten Thermometriedaten bzw. Elastizitätsmesswerte/ Viskositätsmesswerte können beim Bearbeiten/Erzeugen der 3D-Polymerstruktur, etwa beim Festlegen der Dauer bzw. der Intensität des zur Anregung eines jeweilig definierten Voxels einzustrahlenden HF-Felds, berücksichtigt werden.

Beim Bearbeiten/Fertigen der 3D-Polymerstruktur stellen thermodynamische Phänomene bekanntlich eine wichtige Artefaktequelle dar, deren Effekte es bestmöglich zu beherrschen gilt. Dies, um sowohl mikrodimensionalen Einbußen der Detailauflösung als auch makrodimensionalen Struktur-Inhomogenitäten und -irregularitäten der 3D-Polymerstruktur vorzubeugen.

Thermosensitive Sequenzen im Rahmen sogenannter MRT-assistierter HIFUS-Behandlungen (hochfokussierter Ultraschall) haben ihre klinische Praktikabilität und Verlässlichkeit zwischenzeitlich bewiesen. Insbesondere die Protonenresonanz-Methode zeichnet sich dabei durch eine hohe räumliche, zeitliche und thermometrische Auflösung und Reliabilität aus und bietet sich daher auch zur Überwachung des Bearbeitungs- bzw. Fertigungsprozesses an, um unvorteilhafte Wärmeableitungen bzw. -kumulationen im dreidimensionalen Raum frühzeitig zu detektieren. Die Vorrichtung kann nach der Erfindung auf die Durchführung solcher thermosensitiven Sequenzen ausgelegt sein.

Die Vorrichtung weist vorzugsweise eine Softwareapplikation auf, mittels derer anhand der im Rahmen der thermosensitiven Sequenzen gewonnenen Daten absolute Temperaturwerte ermittelbar und vorzugsweise farbig codierbar, benutzerdefinierte topographische und thermische Schwellenwerte ortsspezifisch mit Alarmen koppelbar und die Einhaltung präziser Expositionsdosisgrenzen automatisierbar bzw. semiautonom regulierbar sind.

Während eine Verminderung der Detailauflösung beim Bearbeiten bzw. Fertigen der 3D-Polymerstruktur auf Unschärfen der De-/Polymerisationsgrenzen zurückzuführen ist, welche infolge thermodynamischer, voxelüberschreitender Wärmeleitungen während des Fertigens auftreten, demarkieren sich makrodimensionale Aberrationen auf der Basis grober Wärmeakkumulationen vornehmlich erst nach dem Ende der HF-Einstrahlung. Dies kann zu einer Schrumpfung und einen Verzug der bearbeiteten Polymerstruktur bzw. der zu erzeugenden infolge der Materialrelaxation im Rahmen der Abkühlung führen. Diese Phänomene können durch Vorwärmen und Nachwärmen der 3D-Polymerstruktur bzw. des Präpolymers weiter reduziert werden.

Bei der (nicht beanspruchten) in-vivo- Bearbeitung und Fertigung lassen die physiologische Körpertemperatur und die relativ niedrigen Transitionsschwellen der Präpolymere bzw. des (teil-)ausgehärteten Polymermaterials im Vergleich zum industriellen (ex-vivo) Bearbeiten/Fertigen von 3D-Polymerstrukturen ohnehin nur sehr geringe Temperaturgefälle erwarten, welche z.B. über akzessorische Wärmequellen (z.B. Infrarot-Dioden, UV-/Laserdioden, energiereiche Strahler, heiße Luft) bzw. eine fraktionierte Erwärmung, optimiert durch Segmentierungsverfahren und Thermometrieschwellen, zu beherrschen sein sollten.

Ein Anlassen - also Vorwärmen - der 3D-Polymerstruktur/des Präpolymers - ggf. auch des gesamten Situs bzw. - in vivo der anatomischen Region - dient dabei sowohl einer Milderung der Temperaturgradienten und Homogenisierung des Temperaturprofils allgemein, als auch speziell der Reduktion induktiv zu applizierender Energiemengen und somit der Risikoreduktion aberranter Wärmedynamiken auf Mikro- und Makroniveau. Je klarer und enger definiert bei der Bearbeitung und Fertigung des 3D-Polymerstruktur die Transitionsschwelle des Materials ist, desto klarer ist die fertigungstechnische Trennschärfe. Darüber hinaus gilt, je geringer die Wärmeleitfähigkeit des eingesetzten Präpolymers/Polymermaterials, desto geringer die Gefahr einer heterotopen Wärmeakkumulation und somit einer dystopen Polymerisation bzw. Zerstörung des Polymermaterials der 3D-Polymerstruktur, desto höher also das thermische und folglich das strukturelle Auflösungsvermögen.

Um makrodimensionale wie auch mikrodimensionale Wärmeakkumulationen zu vermeiden, sollte die Generierung großer Soliditäts- und Volumendifferenzen, konzentrierter Materialmassen, starker Kalibersprünge und starker Temperaturdifferenzen in der 3D-Polymerstruktur vermieden werden. Andererseits steigt im Präpolymer/in der 3D-Polymerstruktur das bearbeitungstechnische bzw. fertigungstechnische Auflösungsvermögen mit der Steilheit des Temperaturgradienten zwischen induktiv erwärmten, isolierten Einzelvoxeln relativ zu ihrer Umgebung, weshalb an relevanten Grenzzonen, funktionellen Reliefs und Randkanten sogar kühlende Maßnahmen zur Detailoptimierung zu erwägen sind.

Auch eine gewisse Stimulationsredundanz der Oszillatoren bzw. Trägheit der Polymerisation/Depolymerisation (Zerstörung) kann die thermische Artefaktanfälligkeit zugunsten des strukturellen Auflösungsvermögens reduzieren. Beim Multi-Shot-Konzept sind entsprechend mehrere stimulative HF-Impulse notwendig, um die jeweilige Transitionstemperatur der Depolymerisation/Polymerisation zu erreichen, streng abgestimmt auf die thermische Leitfähigkeit des Polymers/Präpolymers, resultierend in einem steileren Temperaturgefälle zum jeweiligen Nachbarvoxel und somit einer höheren Trennschärfe.

Bei der Fertigung der Polymerstruktur verändern sich mit zunehmender Aushärtung des Polymers die strukturellen Gegebenheiten mit erheblichem Einfluss auf die Wärmeableitung. Durch zonal variierende Materialkontinuität und -Solidifikation ergibt sich mit fortschreitender Aushärtung der Polymervorstufe bzw. Destruktion des Polymermaterials ein zunehmend heterogenes thermisches System, in welchem durch Kumulationseffekte ein Nebeneinander von überwärmten und unterkühlten Zonen entsteht, die prospektiv einkalkuliert und proaktiv kompensiert werden müssen, um dystope Polymerisationen/Depolymerisationen (= Zerstörungen) bestmöglich zu vermeiden. Dies gelingt unter Würdigung aller beschriebenen Phänomene und unter Berücksichtigung aller genannten Einflussfaktoren einerseits durch Auswahl eines geeigneten thermoresponsiven (Prä-)Polymer(-Komposits) mit zweckmäßiger Lage der thermischen Transitionsschwellen und vorteilhafter thermischer Leitfähigkeit (s. o.). Insbesondere gelingt dies auch durch gezielte Modulation der HF-Impulsdauer und -Intervalle sowie deren zeitlicher und räumlicher Perzeption im dreidimensionalen Raum durch dynamische Adaptation der resonanzvulnerablen Voxelgröße und -position mittels dynamischer Magnetfeldgradienten.

Eine längere kontinuierliche lokale HF-Anregung des Polymermaterials der zu bearbeitenden 3D-Polymerstruktur /des Präpolymers führt zu einem steileren Temperaturgefälle als eine repetitiv wiederholte kurze, impulsive Anregung, und die sequentielle Anregung zweier unmittelbar benachbarter Voxel führt summarisch zu einer lokal höheren Wärmeakkumulation als die Stimulation zweier voneinander entfernter Voxel.

Zu beachten ist, dass die Polymerisation der Polymervorstufe ggf. das Oszillationsvermögen - und die Resonanzspezifität der (Nano-)Oszillatoren - selbstterminierend einschränken kann. Allgemein gilt: Je dicker und größer die zu erzeugende 3D-Polymerstruktur und je solider ihr Material, desto höher die Gefahr der Wärmeakkumulation; je kleiner, schlanker und diskontinuierlicher die zu erzeugende 3D-Polymerstruktur, desto schneller erfolgt ein Temperaturausgleich. Umso wichtiger ist es, jene hiervon kalkulierbaren Effekte zum frühestmöglichen Zeitpunkt Beachtung zu schenken, sie in die CAD-Konstruktion der zu fertigenden 3D-Polymerstruktur und Optimierung der Sequenzalgorithmik einfließen zu lassen und ggf. in Echtzeit adaptiv zu regulieren.

Hierzu gehört auch die Berücksichtigung spezieller Formen und Strukturunterbrüche als Wärmeleiter bzw. thermische Hindernisse in den CAD-Bauplan, um Wärmeleitungsphänomene zu kanalisieren bzw. einzudämmen, gezielt lokal Spannungen zu erzeugen und andernorts abzubauen.

Unabhängig davon führt eine höhere Konzentration der paramagnetischen Substanz bzw. der (Nano-)Oszillatoren-Konzentration des Polymermaterials der 3D-Polymerstruktur bzw. des Präpolymers theoretisch zu einem höheren induktiven räumlichen Auflösungsvermögen.

Auch eine Steigerung des prozeduralen Umgebungsdrucks, einhergehend mit einer zugleich reduzierten Präpolymerfluktuation können das induktive räumliche Auflösungsvermögen bei der Fertigung der 3D-Polymerstruktur begünstigen. Zwar entstehen Makroaberrationen schichtübergreifend besonders während der Abkühlung, doch auch innerhalb der Schichten bereits bei der Material-Konsolidierung. Auch diesem Phänomen kann durch Erhöhung des Umgebungsdrucks und die Bereitstellung ausreichender Polymervorstufen-Reserven wirksam entgegengewirkt werden.

Mittels der erfindungsgemäßen Vorrichtung ist mithin die 3D-Polymerstruktur in allen Raumrichtungen bearbeitbar bzw. die Polymervorstufe in allen Raumrichtungen polymerisierbar, sodass ein "real 3D"-Bearbeitungs- bzw. Fertigungsverfahren der 3D-Polymerstruktur realisierbar ist. Die traditionellen Schichtungsphänomene werden hier neutralisiert.

Die Steuereinrichtung ist gemäß Anspruch 1 dazu eingerichtet, insbesondere programmiert, alle für das Bearbeiten/Erzeugen der 3D-Polymerstruktur relevanten Betriebsparameter der Vorrichtung anhand von vorgegebenen CAD-Daten der 3D-Polymerstruktur zu steuern.

Gemäß Anspruch 1 ist die Steuerungseinheit dazu eingerichtet, insbesondere programmiert, anhand einer repetitiven Bildgebung im Wege der
- Magnetresonanztomographie,
- der Computertomographie,
- der digitalen Volumentomographie,
- der Sonographie,
- des Laser-Scannings und/oder
- einer Positronen-Emissions-Tomographie
- der Magnetpartikelbildgebung
ermittelte Bilddaten der zumindest teilgefertigten 3D-Polymerstruktur/der Polymervorstufe mit den CAD/CAM Daten der 3D-Polymerstruktur zu vergleichen und bei Überschreiten einer definierten Abweichung der Bilddaten von den CAD/CAM Daten die Betriebsparameter und/oder CAD/CAM-Daten für das Herstellen/Bearbeiten der übrigen 3D-Polymerstruktur derart anzupassen, dass (weiteren) Abweichungen beim Fertigen/Bearbeiten der übrigen 3D-Polymerstruktur von den CAD/CAM Daten entgegengewirkt wird. Mit anderen Worten ist die Steuerungseinheit dazu eingerichtet, den Soll-/Ist Zustand der Polymerisation der Polymervorstufe bzw. der bearbeiteten 3D-Polymerstruktur mittels repetitiver Bildakquisen mit den CAD/CAM Daten abzugleichen und die Steuerung des weiteren Herstellungs- bzw. Bearbeitungsprozesses der 3D-Polymerstruktur auf Grundlage der so gewonnenen Daten fortzusetzen. Optimalerweise bietet sich hierzu die MRI (Magnetresonanztomographie) an, obgleich vorgenannte alternative Bildgebungseinrichtungen - ggf. auch multimodal kombiniert - zum Einsatz kommen können.

Basierend auf der Technik der Resonanz-Nischen Induktion ermöglicht die erfindungsgemäße Vorrichtung somit insgesamt eine Bearbeitung von 3D-Polymerstrukturen, das als MRIA- oder RNI-Δ- Verfahren bezeichnet werden kann sowie bedarfsweise ein additives Fertigungsverfahren der 3D-Polymerstruktur, das als Magnet-Resonanz-Induktions-Polymerisation, oder kurz "MRiP" oder RNI-o, bezeichnet werden kann.

Die mittels der Vorrichtung bearbeitbare bzw. fertigbare 3D-Polymerstruktur kann ein beliebiges Produkt aus einem Polymer- oder Polymerverbundwerkstoff sein. So kann die 3D-Polymerstruktur etwa ein Maschinenelement sein. Hier kommen insbesondere Achsen, Wellen, Lagerelemente, Getriebeteile, Dichtungselemente, Verbindungselemente, Gehäuse(teile) usw. in Betracht. Die 3D-Polymerstruktur kann auch ein Medizinprodukt, beispielsweise eine Epithese, eine Orthese, eine Bandage, eine Zahnspange, eine Zahnverblendung, ein Beatmungsschlauch, ein Gewebekleber, ein medizinisches Implantat oder auch eine (bio-)artifizielle Struktur für den Gewebe- bzw. Organersatz sein. Darüber hinaus kann die 3D-Polymerstruktur ein Alltagsgegenstand, etwa Schmuck, ein Uhrengehäuse, ein Spielzeug, ein Tragebehältnis, Geschirr, Besteck, eine elektrisch isolierende Schicht oder ganz allgemein eine Beschichtung einer beliebigen anderen Struktur sein.

Die (nicht beanspruchte) in-situ-(Bio-)Fabrikation und -Bearbeitung bietet von Anfang an alle Vorteile der natürlichen Geweberegeneration innerhalb eines physiologischen, bioresponsiven Milieus und löst damit die Kardinalprobleme herkömmlicher Gewebeersatzprodukte und konventioneller Bioreaktoren, wie mangelnde Stabilität, mangelnde Integrativität, mangelnde Adaptivität, mangelnde Interaktivität und unzureichende Vitalität. Hieraus folgt, dass eine präzise Stimulation der paramagnetischen Substanz über die Gestaltung der Binnenstruktur und Oberflächentextur der 3D-Polymerstruktur direkten und indirekt Einfluss auf die Qualität und Intensität dessen Wechselwirkungen mit dem Empfängerorganismus nimmt. Wer also die elektromagnetische Induktion beherrscht, beherrscht auch die biomimetische Induktion und dadurch die bioaktive Kompetenz des Implantates und die bioauthentische Kapazität des Regenerates.

Die erfindungsgemäße Vorrichtung birgt den Schlüssel zur Universalität und das enorme Potential, eine 3D-Polymerstruktur zu bearbeiten bzw. sogar herzustellen, die sowohl auf mikrotopographischer als auch makroarchitektonischer Ebene hochflexibel und individualisiert der Diversität therapiebedürftiger Gewebedefekte und -Rezipienten gerecht wird. Mittels der Vorrichtung kann eine 3D-Polymerstruktur bearbeitet und sogar erzeugt werden, die die natürliche Anisotropie hierarchisch organisierter biologischer Gewebe und deterministische Komplexität bioartifizieller Interfaces authentisch nachbildet, um zum frühestmöglichen Zeitpunkt und nachhaltig die biomimetischen Grundlagen für eine langfristige Funktionalität des Implantates im systemischen Gesamtverbund zu schaffen.

Die Vorrichtung kann beispielsweise beim (nicht beanspruchten) in-vivo Herstellen und Bearbeiten der 3D-Polymerstruktur eingesetzt werden. Hier kann eine nahtlose Verknüpfung der zu erzeugenden 3D-Polymerstruktur mit köpereigenen oder körperfremden Strukturen, d.h. deren Verankerung am Zielort mit dem jeweils umliegenden Gewebe, realisiert werden. Damit erlaubt die Vorrichtung ein direktes in-situ-3D-Bioprinting mitsamt (Nach-)Bearbeitung der 3D-Polymerstruktur im lebenden Organismus. Die optionale Vitalisierung der 3D-Polymerstruktur durch passive und/oder aktive Zellbesiedelung kann beispielsweise kontaktlos und minimalinvasiv realisiert werden.

Weist die Vorrichtung zumindest eine oder auch mehrere der vorstehend genannten Einrichtungen für die Bild(daten)akquise (=Bildgewinnungseinrichtung) auf, wird dadurch eine bildgesteuerte bzw. bildnavigierte Applikation oder Positionierung der 3D-Polymerstruktur/Polymervorstufe am vorgegebenen Zielort ermöglicht. Dies ist insbesondere bei der in-vivo Fertigung der 3D-Polymerstruktur aus der Polymervorstufe von Vorteil.

Zu beachten ist, dass die erfindungsgemäße Vorrichtung bei ihrer Verwendung im medizinischen Bereich im weiteren Sinne eine 3D-Bearbeitung bzw. 8D-Fertigung und der 3D-Polymerstruktur ermöglicht. Also einer Technologie, die achsenfrei in allen Raumrichtungen Material "addieren" und "subtrahieren" kann ("real" 3D). Die 3D-Polymerstruktur kann bei der (nicht beanspruchten) in-vivo Fertigung mit ihrer Umgebung interagieren (4D), diese instruieren (5D), durch Zellen vitalisiert werden (6D) und hierdurch wandlungsfähig bis zur vollkommenen biologischen Integration (7D) sein, darüber hinaus aber auch kontaktlos und ggf. mehrzeitig von extern modifiziert werden (8D). Besonders hervorzuheben ist hierbei, dass die "real" 3D-Prozessierung eine homogene isotrope - also uniforme - Belastbarkeit der 3D-Polymerstruktur gewährleistet, wohingegen klassische 3D-Druck-Produkte abhängig von der traditionellen Aufbauachse (z-Achse) in der Regel ein richtungsabhängiges mechanisches Lastaufnahmevermögen aufweisen.

Im medizinischen Kontext dient die erfindungsgemäße Vorrichtung bzw. das erfindungsgemäße 3D-Verfahren (MRiP/MRiA) dem Ziel, die Integrität und Interaktivität funktioneller anatomischer Gesamtgefüge zu reparieren, zu rekonstruieren, zu respektieren, zu korrigieren und zu optimieren, um authentische regenerative Vorgänge zu stimulieren und zu amplifizieren. Dies gilt insbesondere für sehr kleine anatomische Funktionseinheiten und sehr große Gewebevolumina, welche sich bislang einer suffizienten "Restitution ad integrum" mit herkömmlichen Techniken entzogen.

Das Prinzip der kontaktlosen Wärmeerzeugung durch induktive Leistungsübertragung mittels elektromagnetischer Wechselfelder bzw. HF-Strahlung ist allgemein bekannt und findet abseits industrieller Herstellungsprozesse seit Jahrzehnten Anwendung in der physikalischen Medizin. Dass sich durch Kopplung (superpara-)magnetischer Nanopartikel an elektromagnetische Wechselfelder im menschlichen Körper biologisch wirksame und reliabel therapeutisch nutzbare Wärmephänomene erzeugen lassen, belegt nicht zuletzt auch die FDA-Zulassung der onkologischen interstitiellen Hyperthermie.

Eine Instrumentalisierung dieser Methode zum additiven Strukturaufbau bzw. differenzierten Strukturabbau im Sinne einer induktiven Polymerisation thermosensibler Polymer-Komposit-Präparationen zu mehrdimensionalen Strukturen bzw. deren kontaktfreie Bearbeitung wurde jedoch bislang nie beschrieben. Dies mag der Tatsache geschuldet sein, dass eine hochpräzise Fokussierung elektromagnetischer Felder bis heute selbst unter Laborbedingungen nur mit unverhältnismäßig großem technischem Aufwand gelingt. Eine grobe Fokussierung, wie sie sich z.B. mittels frequenzkohärenten Verstärkern erreichen lässt, ist für präzise additive Technologien hingegen nicht ausreichend.

Da die elektromagnetische Induktion im Gegensatz zu den etablierten traditionellen Verfahren der kontaktlosen Energieübertragung (UV-, IR-, Ultraschall, LASER) weder mit zunehmender Laufstrecke (Eindringtiefe) noch an Gewebeübergängen relevante unerwünschte Interferenz- und Absorptionsphänomene zeigt und unter Einhaltung gesetzlicher Dosisgrenzwerte und Frequenzspektren kein biologisch schädliches Potenzial aufweist, keiner Schutzatmosphäre oder starrer, mechanischer Führungssysteme bedarf, ist sie als idealer Energievermittler für die kontaktlose 3D-Bearbeitung und 3D-Fertigung, insbesondere auch bei der in-situ-Bearbeitung bzw. -Biofabrikation, zu betrachten. Die außergewöhnliche Universalität von MRiA (=RNI-Δ) bzw. MRiP (= RNI-o) hat im Bereich der Bearbeitung sowie Fertigung von 3D-Strukturen disruptives Potential.

Die erfindungsgemäße Vorrichtung und auch das Verfahren (MRiP/MRiA) zum Bearbeiten bzw. zum Erzeugen der 3D-Polymerstruktur bieten nun erstmals einen praktikablen Lösungsansatz, wie induktive Energiedepositionen auch mittels ungerichteter elektromagnetischer Wechselfelder (HF-Feld) ortsspezifisch realisiert, moduliert und gezielt zum kontrollierten Strukturabbau bzw. kontrollierten additiven Strukturaufbau genutzt werden können. Dadurch wird eine induktive Fertigung ermöglicht.

Im natürlichen Zustand weisen alle ausreichend kleinen paramagnetischen Substanzen, insbesondere superparamagnetische Nanopartikel, etwa nanopartikuläre Magnetitpartikel, im Sinne von Nano-Oszillatoren ein durch ihr elektromagnetisches Umgebungsmilieu und ihren individuellen Energiegehalt definiertes jeweils eigenes minimales Schwingungsmoment auf. In einem homogenen statischen Magnetfeld, dessen Feldstärke ein Vielfaches der elektromagnetischen Umgebungsmilieus der Oszillatoren beträgt, richten alle Oszillatoren ihre Rotationsmomente parallel bzw. antiparallel zu den Magnetfeldlinien des statischen Feldes aus und sind durch ein äußeres HF-Feld mit einer Frequenz, die der Resonanzfrequenz f₀ der paramagnetischen Oszillatoren entspricht oder im Wesentlichen entspricht, zu thermogenen Oszillationen anregbar.

Die Steilheit und Schnelligkeit der dynamischen Gradientenfelder der Vorrichtung ist in mind. 3 Raumrichtungen vorgebbar, um die jeweilige Voxel-Dimension (räumlich/zeitlich) und die Voxel-Verteilung (räumlich/zeitlich) zu definieren.

Nach der Erfindung kann der Arbeitsbereich der Vorrichtung innerhalb einer Umhausung bzw. eines Gehäuses der Vorrichtung angeordnet sein. Dadurch kann eine definierte Arbeitsumgebung für das Bearbeiten/Erzeugen der 3D-Polymerstruktur bereitgestellt und aufrechterhalten werden. So können beispielsweise die Temperatur, die Zusammensetzung der Atmosphäre (=Arbeitsatmosphäre), der Atmosphärendruck im Arbeitsbereich sowie auch die Feuchtigkeit der den Arbeitsbereich unmittelbar umgebenden Arbeitsatmosphäre vereinfacht und kostengünstig dem Bedarf entsprechend eingestellt werden. Die Umhausung kann aus Kunststoff, beispielsweise in Form einer Kunststofffolie, aus Glas oder aus einem anderen MRI-geeigneten Material bestehen.

Besonders bevorzugt weist die Vorrichtung eine Pumpe auf, mittels derer der Arbeitsbereich mit einer für den jeweiligen Fertigungsprozess vorgegebenen Arbeitsatmosphäre befüllbar bzw. im Arbeitsbereich ein subatmosphärischer Druck bzw. ein näherungsweises Vakuum aufbaubar ist.

Die Vorrichtung kann nach der Erfindung eine Temperiereinrichtung zum Temperieren des Arbeitsbereichs bzw. der im Arbeitsbereich angeordneten 3D-Polymerstruktur/Polymervorstufe aufweisen. Mittels der Temperiereinrichtung kann die 3D-Polymerstruktur/Polymervorstufe bedarfsweise gekühlt werden. Dadurch kann beispielsweise vorab und/oder während der Herstellung der 3D-Polymerstruktur einer unerwünschten unkontrollierten Polymerisation der Polymervorstufe außerhalb des zur Polymerisation ausgewählten/bestimmten Voxels entgegengewirkt werden. Mittels der Temperiereinrichtung kann der Arbeitsbereich bzw. die darin angeordnete 3D-Polymerstruktur/Polymervorstufe bei Bedarf aber auch aufgeheizt werden. Diese können dadurch "angelassen", werden, um deren jeweilige differenzierte Zerstörung (=Depolymerisation) bzw. Polymerisation zu begünstigen.

### Polymervorstufe

Die Erfindung bezieht sich auch auf eine (nicht beanspruchte) Polymervorstufe mit zumindest einer paramagnetischen Substanz zum Fertigen (=Erzeugen) der 3D-Polymerstruktur. Die Polymervorstufe umfasst nach der Erfindung Monomere und/oder Oligomere und/oder Polymere, die im Wege einer thermischen Polymerisation, d. h. durch Einwirken von thermischer Energie, polymerisieren.

Die Polymervorstufe und damit die 3D-Polymerstruktur kann insbesondere sogenannte Biopolymere umfassen. Diese zeichnen sich durch eine hohe Biokompatibilität, hohe Bioaktivität und Zellbindungskapazität aus. Nach der Erfindung kommen hier beispielsweise Polysaccharide, Glykosaminoglykane, Polypeptide und/oder Proteine in Betracht. Insbesondere Alginate, Hyaluron, Kollagene/Gelatine, Chitosan, Fibrin, Seidenfibroin, Cellulose und selbst Derivate der Extrazellulärmatrix des Menschen (ECM-Derivate) und sogenannte (bio)artifiziellen Polymere sind vorstellbar. Auch Biopolymere, etwa marine Kollagene, etwa aus Fischabfällen (Fisch-Gelatine-Metacrolyl = FGelMa), sind vorstellbar.

Artifizielle Polymere wiederum weisen eine hohe mechanische Stabilität und präzise modulierbare Eigenschaften (z.B. definierte Abbaugeschwindigkeit) auf. Hierzu zählen traditionelle Werkstoffe der Biotechnologie + Pharmakologie, aber auch in zunehmendem Maße etablierte Substanzen der kunststoffverarbeitenden Industrie, von denen nachstehend einige geeignete genannt sind:
PLA (Poly-Milchsäure), PEG (Polyethylenglykol), PCL (Polycaprolacton; ein sehr guter Ausgangsstoff für anorganische Biokeramiken), PGA (Polyglykolsäure), PLGA (Poly(-lactid-co-glycolid), PEO (Polyethylenglycol), PPO (Polyphenylenoxid), PU (Polyurethan), PEEK (Polyetheretherketon), Polyamide (Nylon; +/- Polyester), PCU-Sil (Polycarbonat-basierte Urethan-Silikone), PUU (Poly-Urea-Urethane), SMP (shape-memory-Polymere), Acrylnitrile (z.B. ABS: Acrylonitril-Butadien-Styrol), Block-Copolymere, Flüssig-Kristall-Polymere.

Um die Vorteile beider Gruppen zu nutzen, kann die Polymervorstufe/3D-Polymerstruktur nach der Erfindung einen Multi-Material-Mix (z.B. PEG-Kollagen-Hydrogele) umfassen.

Die paramagnetische Substanz umfasst vorzugsweise paramagnetische Partikel, insbesondere in Form paramagnetischer Mikro- oder Nanopartikel. Die paramagnetische Substanz ist vorzugsweise hochspezifisch frequenzselektiv, d. h. durch Einstrahlung eines HF-Feldes mit definierter Frequenz bzw. einem definierten Frequenzband zu Oszillationsbewegungen anregbar. Durch diese Oszillationsbewegungen der paramagnetischen Substanz kann ein thermogener Energieeintrag in die Polymervorstufe zur Polymerisation der Polymervorstufe erreicht werden.

Die Konzentration der vorgenannten Partikel beträgt vorzugsweise > 1000 Partikel pro Kubikmillimeter der Polymervorstufe, insbesondere > 10.000 Partikel pro Kubikmillimeter der Polymervorstufe. Dadurch ist während des Fertigungsprozesses eine zuverlässige und homogene Polymerisation der Polymervorstufe im jeweilig ortskodierten Voxel der Polymervorstufe gewährleistet. Die Konzentration der Partikel kann in Abhängigkeit von der aus der Polymervorstufe zu erzeugenden 3D-Polymerstruktur bzw. der Zusammensetzung der Polymervorstufe dem Bedarf entsprechend eingestellt sein. Nach der Erfindung kann die Konzentration der Partikel bis zu 10¹⁷ Partikel pro Kubikmillimeter der Polymervorstufe betragen. Dadurch können auch kleinste 3D-Polymerstrukturen und mit einer bislang unerreichten Detailauflösung additiv gefertigt werden.

Die paramagnetische Substanz umfasst nach der Erfindung vorzugsweise Metallpartikel. Die Partikel können beispielsweise aus Magnetit (Fe₃O₄) oder einem Silberhalogenid (AgₙXₙ) bestehen.

Die paramagnetische Substanz ist nach einer Ausführungsform der Erfindung vorzugsweise in der Polymervorstufe suspendiert. Eine möglichst homogene Verteilung/Suspension der paramagnetischen Partikel in der Polymervorstufe ist vorteilhaft.

Nach einer alternativen Weiterbildung der Erfindung kann die paramagnetische Substanz auch zumindest teilweise oder vollständig an Monomere/Polymere der Polymervorstufe gebunden sein. Derlei Metallorganyle bzw. metallorganische Verbindungen weisen in der Regel eine polare kovalente Bindung zwischen einem Kohlenstoff-Atom und mindestens einem Metall- oder elektropositiven Elementatom auf.

Nach einer besonders bevorzugten Weiterbildung der Erfindung können sich die paramagnetischen Partikel der Polymervorstufe/3D-Polymerstruktur zumindest teilweise in ihren Materialeigenschaften bzw. in ihrer spezifischen chemischen Zusammensetzung und/oder Größe voneinander unterscheiden.

Im Hinblick auf die Biokompatibilität, die Transduktivität bzw. Thermogenität der Partikel können diese jeweils eine (vorgegebene) Beschichtung aufweisen. Die Beschichtung der Partikel kann beispielweise Titan umfassen. Denkbar sind auch andere biokompatible Beschichtungsmaterialien, wie etwa Polyetheretherketon (PEEK), Polyetherimid (PEI), Polycarbonate, Acrylnitril-Butadien-Styrol, Polylactide (PLA), Polyhydroxyessigsäure, Polyglycolsäure.

Die Polymervorstufe/3D-Polymerstruktur kann/können darüber hinaus (zusätzlich zur paramagnetischen Substanz) erfindungsgemäß einen oder mehrere Zuschlagstoffe (=Additiva) umfassen. Die Zuschlagstoffe können organische und/oder anorganische Zuschlagstoffe sein.

Die Kapazität optionaler biologischer, chemischer, physikalischer und pharmazeutischer Additiva sollte keinesfalls unterschätzt werden. Diese dienen beispielsweise als Induktoren, Promotoren, Katalysatoren und Terminatoren der (bio-)chemischen Reaktivität, der Homogenisierung und Stabilisierung des Milieus bzw. der Resonanzbedingungen und somit auch der Artefaktreduktion. Gleichermaßen können sie zu einer Erhöhung als auch zur Minderung der elektrischen bzw. thermischen Leitfähigkeit bzw. Isolierung innerhalb der Polymervorstufe führen und hierdurch die Polymerisation positiv oder negativ beeinflussen, sensible internale und externale Zonen protegieren und ggf. das Post-Processing vereinfachen.

Insbesondere Nanopartikeln gelingt es in kleinesten Mengen die originären Eigenschaften von Werkstoffen und ihren Endprodukten dramatisch zu verändern, diese zu funktionalisieren und zu (bio-)aktivieren (z.B. Au, Ag, Montomorillonite, Laponite, Hectorite, Silica, Fe2O3, Fe3O4, Graphene, GraphenOxide, Nanocellulose, LDHs (layered double hydroxides, Pyrrole, ...).

Die Additiva können nach einer Weiterbildung als Adsorbantien fungieren, sei es zur Bindung oder Umwandlung toxischer oder kontraproduktiver Meta- und Kataboliten, unerwünschter Botenstoffe oder Schmerzmediatoren, um z.B. immunologische Abwehr- und Sensibilisierungskaskaden oder Infektionen zu verhindern.

Geeignete Additiva können es erlauben, die rheologischen Eigenschaften, u.a. die Oberflächenspannung der Polymervorstufe, zu beeinflussen, um deren Injektabilität und Mikroadhäsivität zu maximieren.

Andere geeignete Additiva wiederum können helfen, die Endkonsistenz der 3D-Polymerstruktur gezielt zu modulieren bzw. zu limitieren (z.B. Füllstoffe). Gleichermaßen erwähnenswert sind Zuschlagstoffe in Form von Biologika zur Zellaktivierung und Steuerung wie Wachstumsfaktoren und Immunmodulatoren (z.B. Interferone), Radiomodulatoren zur Unterstützung von Strahlentherapien und Zytostatika, aber auch Visualisierungshilfen wie Kontrastmittel oder andere kontaktlos von extern detektierbare und quantifizierbare Marker.

Die Zuschlagstoffe können mithin insbesondere aus der Gruppe der
- Fasern,
- Farbstoffe,
- antibakteriellen Substanzen,
- Wachstumsfaktoren
- Nanopartikel/-tubes,
- mineralischen Füllstoffe (z.B. Tricalcium-Phosphat-Zement, Nano-Hydroxyapatit, bioactive glass),
- metallischen Werkstoffe (z.B. Silber, Gold, Magnetite (Fe2O3, Fe3O4) - z.B. SPION = superparamagnetische Eisenoxidnanopartikel, Gd-Chelate/Konjugate),
- Glykosaminoglykane,
- sogenannten MMC-Stoffe (zum sogenannten macromolecular crowding; z.B. Dextrane oder Ficol (= Saccharose-Epichorhydrin-Copolymer))
- Polypeptid-Motive wie -RGD-Sequenzen (Arginin, Glycin und Asparaginsäure), die beispielweise in Proteinen der extrazellulären Matrix vorkommen (z.B. in Fibronectin und Vitronektin),
- -IKVAV + - YIGSR (Laminin) und
- Promotoren
- Terminatoren,
- Inhibitoren,
- Katalysatoren,
- Sensitizer,
- Immunmodulatoren (wie VGF oder das Molekül JNK3)
sein.

Die vorgenannten Zuschlagstoffe können frei gelöst, substanzgebunden, oder temporär fixiert an Transportmoleküle bzw. in Thermosomen vorliegen.

Die Polymervorstufe kann insbesondere eine liquide Form oder Gelform aufweisen. Besonders bevorzugt weist die Polymervorstufe eine Viskosität auf, die zwischen 10² und 10⁶ mPa·s beträgt.

Nach der Erfindung kann die Polymervorstufe auch in Form eines sogenannten "Squids" vorliegen. Derlei Squids sind anderweitig präformierte, unvollständig ausgehärtete Polymervorstufen.

Nach einer bevorzugten Weiterbildung der Erfindung ist die Polymervorstufe für den Menschen nicht-toxisch. Dadurch kann die Polymervorstufe im menschlichen Körper für die in-vivo-3D-Fertigung eingesetzt werden.

Ganz besonders bevorzugt ist die Polymervorstufe im nicht-auspolymerisierten Zustand im menschlichen und/oder tierischen Körper, beispielsweise durch körpereigene Enzyme, abbaubar.

### MRiA-Verfahren (=RNI-Δ-Verfahren)

Das erfindungsgemäße Verfahren zum Bearbeiten der 3D-Polymerstruktur weist die Schritte a-e gemäß Anspruch 5 auf.

Nach einer besonders bevorzugten Weiterbildung der Erfindung umfasst das Verfahren zum Bearbeiten der 3D-Polymerstruktur die folgenden weiteren Schritte, die dem Bereitstellen der 3D-Struktur vorzugsweise vorgeschaltet sind:
g. Anordnen einer Polymervorstufe, die eine, bevorzugt homogen verteilte, paramagnetische Substanz umfasst, im Arbeitsbereich der Vorrichtung;
h. Ortskodieren eines Voxels V innerhalb der Polymervorstufe in Abhängigkeit von den CAD/CAM Daten durch Anlegen magnetischer Gradientenfelder B₁, B₂, B₃ in mindestens allen drei Raumrichtungen x, y, z;
i. Polymerisieren der Polymervorstufe, bevorzugt alleinig, in dem zumindest einen ortskodierten Voxel V durch Einstrahlen von HF-Strahlung mittels derer die paramagnetische Substanz in dem jeweiligen weiteren Voxel V zu Oszillationen angeregt wird; und
j. nachfolgend sequentielles Ortskodieren weiterer, bevorzugt räumlich jeweils aneinander angrenzender, Voxel V in der Polymervorstufe in Abhängigkeit von den CAD/CAM-Daten und
k. Polymerisieren der Polymervorstufe in den jeweilig weiteren ortscodierten Voxeln V durch Einstrahlen von HF-Strahlung, mittels derer die paramagnetische Substanz in dem jeweiligen weiteren Voxel V zu thermogenen Oszillationen angeregt wird.

Bei der letztgenannten Ausführungsform handelt es sich mithin um ein kombiniertes Fertigungs- bzw. Herstellungs- sowie Bearbeitungsverfahren.

Zu beachten ist, dass sich die vorstehend erläuterten Bearbeitungsschritte und die polymerisationsbezogenen Schritte, ggf. iterativ, abwechseln können. Dadurch ist die Fertigung selbst komplexester 3D-Strukturen möglich.

Bei dem erfindungsgemäßen Verfahren beruht die dreidimensionale Ortsauflösung auf den induktiven Magnetresonanzphänomenen eines Wechselspiels aus elektromagnetischer Resonanzisolation und Resonanzstimulation. Zwar nutzen diagnostische bzw. analytische MR-Verfahren diese Strategie. Allerdings wurden bislang keine spezifisch resonanzfrequenzselektiven, thermogenen, paramagnetischen Nanopartikel eingesetzt, die durch HF-Anregung in eine thermogene Oszillation versetzt werden und so eine lokal auf das ortskodierte Voxel der Polymervorstufe begrenzte Zerstörung der 3D-Polymerstruktur bzw. Polymerisierung der Polymervorstufe, d.h. Induktion chemischer Bindungen in der thermosensitiven Polymervorstufe, induzieren.

Das hier vorgeschlagene Verfahren erlaubt eine kontinuierliche Volumen-Graduierung der Zerstörung der 3D-Polymerstruktur bzw. Polymerisation (kleiner, gleich und größer einer herkömmlichen Schichtdimension) der Polymervorstufe, also eine sowohl hochpräzise punktuelle als auch "en bloc" Zerstörung der 3D-Struktur bzw. Polymerisation und damit Aushärten der zu erzeugenden 3D-Polymerstruktur.

Damit wird ein effizientes 3D-Bearbeiten und -Fertigen beliebiger, insbesondere bio-artifizieller Strukturen, ermöglicht.

Die präzise HF-Stimulation frequenzselektiver thermogener Nanooszillatoren in elektromagnetischen Resonanznischen, welche ortsspezifisch, vorzugsweise auch auf der Basis hochauflösender Bilddaten, durch gezielte Überlagerung magnetischer Gradientenfelder innerhalb der zumindest teilpolymerisierten/auspolymerisierten 3D-Polymerstruktur bzw. einer mehr oder weniger liquiden thermosensitiven Polymervorstufe erzeugt werden, ermöglicht wie bereits dargelegt, das Bearbeiten und Fertigung von 3D-Polymerstrukturen jedweder Geometrie, Konfiguration und Komplexität - in Abhängigkeit der eingesetzten Materialien auch jeglicher Konsistenz - individuell in vitro. Die Strategie, eine 3D-Polymerstruktur durch Abbau kleinstmöglicher Untereinheiten zu bearbeiten bzw. aus kleinstmöglichen Untereinheiten aufzubauen, resultiert in einer maximalen Gestaltungsfreiheit und Adaptabilität.

Dies in einer bis dato unerreichten Detailtreue und Geschwindigkeit. Das Verfahren kann für das Bearbeiten und die Fertigen von Gebrauchsgegenständen, Maschinenelementen, medizinischen Produkten, insbesondere bei der medizinischen und biotechnologischen Biofabrikation, und sogar beim in-situ-Bioprinting neue Wege ermöglichen. Dies insbesondere auch deshalb, weil das erfindungsgemäße Verfahren (MRiP) eine präzise Oberflächengestaltung und eine nahtlose Verknüpfung bzw. Verankerung mit anderen Strukturen ermöglicht. Dadurch kann bei Implantation der 3D-Polymerstruktur eine vereinfachte und zuverlässigere Vitalisierung durch passive/aktive Zellbesiedelung begünstigt werden. Im medizinischen Kontext kann MRiP/MRiA ermöglichen, Implantate zu erzeugen, die die Integrität und Interaktivität funktioneller anatomischer Gesamtgefüge reparieren, rekonstruieren, respektieren, korrigieren und optimieren, um natürliche regenerative Vorgänge zu stimulieren und zu amplifizieren.

Eine grobe Fokussierung, wie sie sich z.B. mittels frequenzkohärenten Verstärkern erreichen lässt, ist für präzise subtraktive/additive Technologien nicht ausreichend. Die elektromagnetische Induktion zeigt im Gegensatz zu den etablierten traditionellen Verfahren der kontaktlosen Energieübertragung mittels UV-/IR-Strahlung, Ultraschall oder LASER weder mit zunehmender Laufstrecke in der 3D-Polymerstruktur/Polymervorstufe bzw. im Gewebe (Eindringtiefe) noch an Materialübergängen relevante unerwünschte Interferenz- und Absorptionsphänomene. Darüber hinaus weist die elektromagnetische Induktion unter Einhaltung gesetzlicher Dosisgrenzwerte und Frequenzspektren kein biologisch schädliches Potenzial auf, bedarf per se keiner Schutzatmosphäre oder starrer, mechanischer Führungssysteme und ist deshalb als ein idealer Energievermittler für die kontaktlose in vitro Biofabrikation zu betrachten.

Das erfindungsgemäße Verfahren bietet nun erstmals einen praktikablen Lösungsansatz, wie induktive Energiedepositionen mittels ungerichteter elektromagnetischer Wechselfelder (HF-Feld) ortsspezifisch realisiert, moduliert und gezielt zum kontrollierten subtraktiven Strukturabbau sowie zum additiven Strukturaufbau genutzt werden können (= "induktive Manufacturing")

Im natürlichen Zustand weisen alle superparamagnetischen Oszillatoren ein durch ihr elektromagnetisches Umgebungsmilieu und ihren individuellen Energiegehalt definiertes jeweils eigenes minimales Schwingungsmoment auf. In einem homogenen statischen Magnetfeld, dessen Feldstärke ein Vielfaches der elektromagnetischen Umgebungsmilieus der paramagnetischen Oszillatoren beträgt, richten alle Oszillatoren ihre Rotationsmomente parallel bzw. antiparallel zu den Magnetfeldlinien des statischen B₀-Felds aus, einhergehend mit einer Synchronisierung ihrer Resonanzfrequenzempfänglichkeit für Wechselfeld-Impulse, welche - neben Material, Polarität und Konfiguration der Oszillatoren - proportional von der Feldstärke des homogenen Magnetfelds abhängt. Dies bedeutet, dass für jede Feldstärke optimalerweise nur eine einzige, spezifische Frequenz existiert, welche alle entsprechend konfektionierten Oszillatoren maximal stimuliert - die sogenannte Resonanzfrequenz. Unter Resonanzbedingungen weisen also alle empfänglichen Oszillatoren eine maximale Energieabsorptionsbereitschaft hinsichtlich des spezifischen Hochfrequenz-Wechselfeldes auf, resultierend in einer signifikant erhöhten Oszillationsbewegung, welche aufgrund von Widerstands- und Reibungseffekten mit minimalem Kopplungsabstand in thermische Energie umgewandelt wird. Wie in der Kernspintomographie zur Schichtselektion und Ortscodierung dienen MRiA/MRiP dynamische Gradientenfelder in mindestens 3 Raumrichtungen zur "indirekten Fokussierung" dieser Hochfrequenz-Impulse. Diese Magnetfelder sind durch einen kontinuierlichen Anstieg bzw. Abfall der jeweiligen Magnetfeldstärke entlang ihrer charakteristischen Achsen [x, y, z], relativ zum statischen permanenten Magnetfeld und der im Arbeitsbereich den Magnetfeldern ausgesetzten Polymervorstufe gekennzeichnet.

Ein durch spezielle Spulen zugeschaltetes Gradientenfeld in der x-Achse überlagert also das zuvor homogene permanente statische B₀-Feld und führt somit zu einem linearen Anstieg bzw. Abfall der statischen Gesamtfeldstärke entlang der x-Achse. Soweit technisch realisierbar, wären steilere nicht-lineare Gradienten vorteilhaft, um die Differenzierung von Nachbar-Voxeln zu verstärken. Gleiches gilt für jeweils zugeschaltete Gradientenfelder entlang der y-Achse und z-Achse, mit der Folge, dass jedes definierte Voxel des Gesamtvolumens des Arbeitsbereichs und damit der 3D-Polymerstruktur/Polymervorstufe bzw. theoretisch jeder Oszillator im dreidimensionalen Raum über seine individuelle, ihm eigene elektromagnetische Nische im Schnittpunkt der mindestens 3 Gradientenfelder verfügt.

Unterscheidet sich also das magnetische Mikromilieu jedes Oszillators - bzw. Voxels - in allen Raumrichtungen linear vom magnetischen Mikromilieu seines Nachbaroszillators -bzw. Voxels -, dann verfügt jeder Einzeloszillator - bzw. jedes Voxel - über seine ganz eigene individuelle Resonanzfrequenz und lässt sich folglich individuell isoliert adressieren und selektiv anregen. Hierbei definiert die Steilheit der Gradientenfelder die Kantenlängen der Voxel in allen 3 Raumrichtungen, optimalerweise durch simultane summarische Überlagerung ihrer lokalen Feldstärke, alternativ sequentiell, z.B. unter Verwendung der inversen Fourier-Transformation. Im Unterschied zu allen herkömmlichen subtraktiven/additiven Verfahren lassen sich auf diese Weise entweder Einzel-Voxel als auch zusammenhängende Volumeneinheiten im Sinne eines multi-Voxel processings "en bloc" polymerisieren.

Anstelle einer einzigen externen Wärmequelle stellt also jeder Nano-Oszillator in sich eine autonome Wärmequelle (mit minimalem Kopplungsabstand) dar, deren Wirkung ohne adressierende Gradientenfelder in einem dreidimensionalen thermischen gitternetzförmigen Erwärmungsmuster aus zunächst multiplen isomorphen und isothermen Hitzeinseln mit Temperaturmaxima jeweils unmittelbar um jeden einzelnen Oszillator resultieren würde. Die charakteristische Leistungsaufnahme der Oszillatoren, ihr Wirkungsgrad und die materialspezifische Wärmeleitung der 3D-Polymerstruktur/Polymervorstufe definieren in einem ansonsten geschlossenen, perfekten System die Dimension, Dynamik und Dauer der Wärmeausbreitung bis zu jenem Zeitpunkt, an welchem eine global homogene Erwärmung des Gesamtvolumens erreicht ist, abhängig von Impulsintensität und Impulsperiodik, welche fortwährend in Echtzeit vom Anwender gesteuert werden können.

In der Praxis führen Wärmeleitungsphänomene zu einem zentrifugalen Temperaturabfall um jeden stimulierten Oszillator, entsprechend einer thermodynamischen, von den Umgebungsbedingungen abhängigen Penumbra. Diese thermischen Inseln neigen dazu, mit zunehmender Anregung über die Zeit zu konfluieren, sich auszugleichen oder gegenseitig zu verstärken.

Unter proaktiver Nutzung der beschriebenen Gesetzmäßigkeiten und nachfolgend aufgeführten Einflussfaktoren können diese Effekte vom Anwender durch fokussierte Stimulation einzelner individueller Oszillatoren(gruppen) isoliert lokal verstärkt oder reduziert werden, um punktuell bzw. zonal die finalen Wärmemuster und konsekutiv die Detailschärfe und Zieleigenschaften des Produktes (z.B. Funktionalität, Haltbarkeit) zu modulieren.

Differenzen der Resonanzempfänglichkeit erlauben also durch kontrollierte Variation der räumlichen und zeitlichen Resonanzbedingungen eine gezielte ortsspezifische Anregung thermogener Nanooszillatoren (= paramagnetische Substanz) (in vitro wie in vivo) und folglich eine hochpräzise Bearbeitung sowie auch Fertigung komplexer 3D-Polymerstrukturen auf der Basis differenzierter Energieabsorptionsmuster und ortsspezifisch reproduzierbarer Temperaturprofile. Die thermischen Effekte elektromagnetisch induktiv stimulierter Nanooszillatoren erlauben dabei neben der Modulation der internalen Polymer-Kohäsion, insbesondere auch eine Steuerung der Adhäsion der 3D-Produkte durch mikroskopische Strukturverzahnung (Mikroadhäsion) an fremden Oberflächen - eine primär zweckmäßige Rheologie der Polymervorstufe vorausgesetzt.

Bei Implantaten können diese in der Übergangszone zum Organismus (Interface) ggf. durch diskontinuierliche thermokoagulatorische Klebeeffekte ergänzt werden, woraus sich optimaler Weise eine kontinuierliche, nahtlose regenerative Einheilung ergibt. In vivo kann dies durch postentzündliche reparative bzw. postnekrotische fokale, interkonnektive Narbenbildungen flankiert werden. Darüber hinaus können ortsspezifische induktive Effekte das Implantatbett - also den Wundgrund - zuvor in förderlicher Weise konditionieren, z.B. durch thermisches Mikrodebridement, Blutstillung und Denervierung.

Der besondere Reiz des erfindungsgemäßen Verfahrens liegt dabei in der subtilen, multiparametrischen Steuerbarkeit der schrittweisen Abbau- bzw. Aufbauphasen der Produktstruktur in Echtzeit, resultierend in einer maximalen Prozesskontrolle, Individualisierbarkeit und Ergebnisqualität.

Grundvoraussetzung hierfür ist eine differenzierte prädiktive Kalkulation der in allen Raumrichtungen zu applizierenden Energiedosen zur temperaturabhängigen Materialzerstörung bzw. Materialsolidifizierung. Die Modulation der pro Volumeneinheit übertragenen Energiedosis gelingt hierbei vor allem durch die zielorientierte Anpassung der elektromagnetischen Induktionsparameter und temporospatiale algorithmische Variation der Resonanzbedingungen, welche sich aus dem Zusammenspiel des statischen mit den dynamischen Magnetfeldern ergibt.

Hierbei gelingt es ungerichteten HF-Stimulationsimpulsen - beispielhaft mit einer Wellenlänge im mehrstelligen Zentimeterbereich (MRT-Diagnostik) - problemlos, selektiv Voxel im Submillimeterbereich anzuregen, ohne relevante Schwächung der eingestrahlten elektromagnetischen Energie durch Laufstrecke, Grenzflächen oder Phasenübergänge.

Gemäß einer ersten Ausführungsform der Erfindung kann die ResonanzEmpfänglichkeit jedes individuellen Nano-Oszillators sequentiell durch eine gezielte Variation der Gradientensteilheit und -Stärke an eine immerzu gleichbleibende Anregungsfrequenz bei allzeit frequenzkonstantem Stimulationsimpuls angepasst werden.

Gemäß einer alternativen Ausführungsform kann die Frequenz des Stimulationsimpulses sequentiell gezielt variabel an eine durch allzeit konstante Gradienteneigenschaften gleichbleibende, individualisierte ResonanzEmpfänglichkeit eines jedes Voxels angepasst sein.

Während die erstgenannte Ausführungsvariante seit Jahrzehnten erfolgreich in der Ortscodierung der diagnostischen Kernspintomographie zum Einsatz kommt, finden Frequenzmodulationstechnologien Anwendung in der industriellen induktiven Metall- und Kunststoffverarbeitung, dies allerdings ohne den Einsatz adressierender Gradientenfelder. Die technische Ausführungsvariante 2 ist zum heutigen Zeitpunkt technisch noch sehr aufwändig.

Zum Erzeugen des B₀-Felds, das der Gleichrichtung der paramagnetischen Substanz d. h. der paramagnetischen Nano-Oszillatoren parallel bzw. antiparallel zu den Magnetfeldlinien sowie der Gleichschaltung ihrer feldstärkenabhängigen Empfänglichkeit für ihren charakteristischen, resonanzinduktiven Hochfrequenzimpuls dient, kann ein Permanentmagnet, ein supraleitender Magnet, ein Elektromagnet oder ein Widerstandsmagnet eingesetzt werden. Entsprechend kann die Vorrichtung einen in der vorstehenden Weise ausgeführten Magneten aufweisen. Die Feldstärke des (statischen) B₀-Felds kann nach der Erfindung ≤ 3 Tesla oder auch ≥ 3 Tesla sein. Ganz besonders bevorzugt ist die Feldstärke des B₀-Feldes > 10 Tesla. Gleichwohl die Magnetresonanztomographie bezüglich der Bild(daten)gewinnung bzw. bildmorphologischen Überwachung des Fertigungsprozesses als Goldstandard anzusehen ist, kann die Feldstärke des B₀-Felds nach der Erfindung im mT-Bereich liegen oder größer sein.

Die Gradientenfelder zur ortscodierten Adressierung der paramagnetischen Substanz der einzelnen Voxel werden mittels Gradientenspulen in ≥ 3 Raumrichtungen generiert. Diese Gradientenfelder werden sequentiell (kurzzeitig) zugeschaltet, wobei deren Feldstärke entlang ihrer jeweiligen Raumachse kontinuierlich ansteigt bzw. abfällt.

Dadurch kann eine ortsspezifische Abschwächung/Verstärkung des statischen B0-Feldes und damit eine Ortscodierung der HF-Empfänglichkeit der paramagnetischen Substanz im dreidimensionalen Raum durch summarische Generierung magnetischer Mikromilieus (Resonanz-Nischen) erreicht werden. Grundsätzlich gilt, je größer die Anstiegsstärke und je kürzer die Anstiegszeit, desto besser die Detailauflösung des Bearbeitungs- bzw. Polymerisationsprozesses. Sofern eine en-bloc Bearbeitung bzw. Polymerisation (also das Polymerisieren mehrerer Voxel bzw. eines größeren Volumes of interest) gewünscht ist, so sind flache Gradienten, also flache Anstiegsstärken vorteilhaft.

Der Hochfrequenz-Feldgenerator (=Hochfrequenz-Einheit) dient der Anregung der paramagnetischen Substanz oder Oszillatoren (unter Resonanzbedingung) mittels HF-Strahlung. Die Hochfrequenzeinheit kann dazu einen HF-Sendeverstärker und zumindest eine HF-Sendespule aufweisen. Die HF-Einheit kann in das Gehäuse der Vorrichtung integriert sein. Alternativ kann die HF-Sendespule in Form einer zum Arbeitsraum der Vorrichtung fei positionierbaren, d. h. mobilen, HF-Spule ausgeführt sein. Hier kommen beispielsweise eine anatomisch-ergonomische Oberflächenspule und eine intrakorporal platzierbare Spule (z. B. Endorektal-Spule oder eine Katheter-montierte Spule) in Betracht. Die Hochfrequenzeinheit kann erfindungsgemäß auch eine Mehrzahl von HF-Spulen in Form von Arrayspulen aufweisen.

Die Wirkdauer der HF-Strahlung ist so niedrig wie möglich "As low as reasonably achievable" (=ALARA) zu wählen, wobei die Ganzkörper SAR-Werte zu beachten sind.

Die Frequenz/Phasenlage/Amplitude der HF-Strahlung ist/wird erfindungsgemäß abgestimmt auf die charakteristische Resonanzfrequenz der anzuregenden (super)paramagnetischen Substanz bzw. Nanopartikel (Oszillatoren) bei gegebener Ziel-Feldstärke der überlagerten Magnetfelder (= statisches Magnetfeld + 3 Gradientenfelder) im jeweiligen Voxel. Ziel ist eine maximale induktive, maximal selektive Anregung der superparamagnetischen Substanz bzw. superparamagnetischen Nanopartikel (Oszillatoren) alleinig eines jeweilig adressierten Voxels unter Resonanzbedingungen.

Die Intervalle zwischen den Einstrahlungen des HF-Impulses (Impuls-Periodik) sind erfindungsgemäß vorzugsweise in Abhängigkeit von den folgenden Faktoren definiert:
- Thermogenität (= charakteristische Leistungsaufnahme + thermischer Wirkungsgrad) jedes Einzeloszillators bzw. deren thermogene Summe/Voxel
- thermische Transistionsschwelle(n) des Polymermaterials der 3D-Polymerstruktur zu zerlegtem Polymermaterial (vgl. z.B. Glasübergangsbereich) bzw. der Polymervorstufe zum (teil)polymerisierten Polymermaterial
- Wärmeleitfähigkeit des Präpolymers bzw. des Polymermaterials
- Zerstörungs- bzw. Polymerisationskinetik (materialspezifisch/architekturspezifisch)
- Zerstörungs- bzw. Polymerisationsmuster (s. CAD); z.B. Auflösungsgrad, Wärmebrücken/-akkumulierende Untereinheiten
   ∘ räumliche Impulsdichte
   ∘ zeitliche Impulsdichte
- (= Modulation des Energieeintrages über temporospatiale ImpulsAlgorithmik)
- ggf. Impulsamplitude/Einstrahlungswinkel bezüglich des anzuregenden Voxels

Die Intervallperiodik bzw. Impuls-Zug-Länge der HF-Stimulation wird primär von den thermodynamischen Effekten im vorgegebenen Setting definiert und ggf. limitiert von der maximalen Geschwindigkeit der Steuerungseinheit + Magnetfeldgradienten, um zwischen 2 präzisen 3D-Voxel-Resonanz-Isolationen ("indirekte Fokussierung") zu wechseln.

Nach der Erfindung
- kann prinzipiell nahezu jedes beliebige Intervall zwischen den Einzelimpulsen der HF-Strahlung liegen, sofern der voxelspezifische bzw. VOI-spezifische (volume of interest) Energieeintrag im Einzelnen bzw. als Summe jeweils den gewünschten thermischen Effekt im Voxel/VOI hervorruft;
- ist bei einer maximal schnellen Voxel- (bzw. VOI-) Resonanz-Isolation (Ziel-Codierung) theoretisch auch eine kontinuierliche HF-Strahlung (HF-Pulsation) möglich, da grundsätzlich nur jene Oszillatoren thermogen schwingen, für welche jeweilig Resonanzbedingungen herrschen;
- ist bei einem (nicht beanspruchten) in-vivo Bearbeiten/Fertigen der 3D-Polymerstruktur eine fraktionierte Impuls-Algorithmik, z.B. mit repetitiven HF-Strahlungs-Zyklen, möglich, die unter thermodynamischen Aspekten vorteilhaft ist und eine Minimierung der globalen HF- und damit Energieeinstrahlung in biologisches Gewebe ermöglicht.

Die Frequenz der in den Arbeitsbereich eingestrahlten HF-Strahlung kann nach der Erfindung grundsätzlich im Kiloherz bis Tetraherzbereich liegen. Besonders bevorzugt beträgt die HF-Frequenz 100 kHz oder 108 Khz bis zu 100 MHz.

Die Steuerungseinheit der Vorrichtung dient folgenden Aufgaben:
a. Anlagensteuerung/Überwachung
b. Datenmanagement
c. CAD-Einheit
d. Bildakquise/-Analyse/-Rekonstruktion

Es versteht sich, dass die Steuerungseinheit eine Applikationssoftware mit KI-Eigenschaften aufweisen kann.

Die Detailgüte der polymeren 3D-Polymerstruktur hängt dabei ab von
- den Eigenschaften der paramagnetischen Substanz/Partikel bzw. (super)paramagnetischen Nanooszillatoren, wie deren
   - Frequenzselektivität
   - charakteristische Leistungsaufnahme
   - thermischem Wirkungsgrad.

Diese Eigenschaften können für die jeweilige paramagnetische Substanz bzw. die Nano-Oszillatoren experimentell bestimmt werden.
- der Konzentration und dem Verteilungsmuster der paramagnetischen Substanz in der Polymervorstufe.
- der Steilheit und Schnelligkeit der mit den Gradientenspulen erzeugten dynamischen Gradientenfelder in den 3 Raumrichtungen;
   - Voxel-Dimension (räumlich/zeitlich)
   - Voxel-Verteilung (räumlich/zeitlich)
- dem Hochfrequenzgenerator bezüglich der
- Spezifität und Homogenität der induktiven HF-Strahlung,
   - Lage
   - Breite
   - Amplitude
   - (frgl. Einstrahlwinkel a relativ zum Hauptmagnetfeld)
- räumlichen und zeitlichen Verteilung der HF-Strahlung (= der HF-Impulse) (Impulsalgorithmik)
- 3D-Wärmemuster
- Zerstörungskinetik/Polymerisationskinetik (z.B. Transitionsschwellen)
- materialspezifische thermodynamische Phänomene
- architekturspezifische thermodynamische Phänomene

Zu berücksichtigen gilt zudem eine abnehmende Detailauflösung des Verfahrens von einer prozeduralen Ebene zur nächsten:
- konstruktives Auflösungsvermögen (CAD)
- elektromagnetisches (induktives) Auflösungsvermögen
- thermisches Auflösungsvermögen
- zeitliches Auflösungsvermögen
- polymeres Auflösungsvermögen
- strukturelles Auflösungsvermögen
- diagnostisches Auflösungsvermögen (Bildgebung, z.B. MRT)
- post-processing Auflösungsvermögen (verfahrensabhängig)

Im klinischen Einsatz bietet in diesem Zusammenhang die Übergangszone des Polymer-Komposits zum Umgebungsgewebe besondere Herausforderungen, da hier signifikante thermische Verluste durch gewebespezifische Wärmeableitungen, Absorptions- und Perfusionsphänomene auftreten - ggf. auch Bewegungsartefakte. Außerdem gilt es relative und absolute thermische Toleranzschwellen biologischer Gewebe zu respektieren.

Limitierender Faktor des Auflösungsvermögens der diagnostischen MRT wird letztlich immer das Signal-Rausch-Verhältnis, also die Zahl der angeregten Protonen bzw. die Summe ihrer auslesbaren, provozierten Echos pro Volumeneinheit relativ zum Umgebungsrauschen bleiben. Dies deshalb, weil die stimulativen Hochfrequenzimpulse der MRT - abgesehen von Vorsättigungsimpulsen - immer alle Protonen einer Schicht bzw. im gesamten präselektierten 3D-Untersuchungsvolumen anregen und die genaue Ortscodierung erst im Ausleseschritt erfolgt. Demgegenüber werden gemäß der Erfindung idealerweise ausschließlich nur jene Oszillatoren präselektierter (=Resonanz-isolierter oder Resonanz-vulnerabler) Voxel der 3D-Polymerstruktur/Polymervorstufe spezifisch angeregt, die gemäß Bauplan als Teil der 3D-Polymerstruktur zerlegt (zerstört) bzw. ausgehärtet werden soll.

Die Gradientenfelder werden also gemäß der Erfindung primär prospektiv aktiviert, wohingegen konventionelle MRT-Sequenzen zunächst mittels nur 1 Gradientenfeld (meist z-Achse) Schicht-Orientierung und -Dicke innerhalb des Untersuchungsvolumens definieren und erst nach dem HF-Impuls zum Zeitpunkt der Echo-Auslese weitere Gradientenechos (meist x-, y-Achse) zur retrospektiven Ortscodierung zugeschaltet werden. Während repetitive Stimulationszyklen für die Analyse der kernspintomographischen Summenechos mittels Fourier-Transformation unerlässlich sind - und zu einem unerwünschten Anstieg der Energieabsorptionsdosen führen - moderiert MRiA/MRiP den Energieeintrag gezielt im Rahmen der temporospatialen Impulsalgorithmik.

Im Allgemeinen sollte die Resonanzfrequenz der Oszillatoren im Herstellungsprozess bewusst ungleich der Protonenresonanzfrequenz gewählt werden, um heterotope Polymerisationen bzw. Depolymerisationen (Polymer-Zerstörungen) durch MRT-Kontrollaufnahmen zu vermeiden. Eine Ausnahme stellen gewisse präformierte "Squids" z. B. 3D-Polymerstrukturen in Form thermoresponsiver Stents/Cages/Cava-Filter, Coils/Occluder, Herzklappen, Gefäß-Endoprothesen, thermoresponsiver Knochenzement bzw. Gewebekleber dar.

Weiterentwicklungen des Verfahrens könnten darüber hinaus von einer Kombination unterschiedlicher Oszillator-Typen mit korrespondierender Frequenzvariation profitieren. Schließlich führen bekanntermaßen hohe Frequenzen zu einem steileren Temperaturanstieg in unmittelbarer Nähe zum Oszillator, niedrige Frequenzen hingegen zu einer sanften, homogenen, globalen Erwärmung der weiteren Umgebung. Entsprechende Effekte könnten sich jedoch auch durch Variation des Einstrahlwinkels a im monofrequenten Impuls-Setting ergeben.

Während sich morphologische Merkmale < 200 µm also bislang in der klinischen MRT-Routine-Diagnostik nicht suffizient abbilden lassen, wird es MRIA/MRiP bzw. mit der erfindungsgemäßen Vorrichtung gelingen, auch um Potenzen kleinere Strukturen (z.B. Poren) subtraktiv zu erzeugen bzw. additiv zu generieren. Dies in einer für die Zellnavigation, Zellkontrolle und histogenetische Determination relevanten Größenordnung zur räumlichen wie zeitlichen Steuerung der Geweberegeneration.

Das Ergebnis der MRiA/MRiP kann nicht zuletzt durch eine repetitive Echt-Zeit-Bildakquise, z.B. auf der Basis robuster MRT-Sequenzen, alternativ auch durch andere Bildgebungseinrichtungen/-verfahren vergleichbarer Ortsauflösung (z.B. Oberflächen-LASER-Scanner, DVT, MPI etc.) weiter verbessert werden.

Nach der Erfindung können für den Bearbeitungsprozess/Fertigungsprozess Voxel in der 3D-Polymerstruktur/Polymervorstufe definiert werden, die jeweils eine einheitliche Größe aufweisen oder die sich in ihrer Größe zumindest teilweise voneinander unterscheiden. Durch das Definieren unterschiedlich großer Voxel bzw. VOI'S (volume of interest) können selbst 3D-Polymerstrukturen mit komplexer Geometrie zügiger bearbeitet bzw. erzeugt werden.

Während des Bearbeitungs- bzw. Fertigungsprozesses kann zumindest ein Teil der auf Grundlage der CAD/CAM-Daten definierten Voxel anhand einer, insbesondere magnetresonanztomografischen, Bildgebung erfassten Bilddaten in ihrer räumlichen Position im Arbeitsbereich, ihrer Größe und/oder in ihrer Geometrie für den weiteren Prozess geändert werden. Dadurch kann die Bearbeitungs- bzw. Fertigungstoleranz der 3D-Polymerstruktur nochmals weiter verbessert werden.

Nach einer Weiterbildung der Erfindung wird der Bearbeitungs- bzw. Fertigungsprozess, bevorzugt intervallweise, unterbrochen, um Bilddaten aus dem Arbeitsbereich, insbesondere von der bereits erzeugten 3D-Polymerstruktur bzw. dem an die 3D-Polymerstruktur angrenzenden Volumen der Polymervorstufe zu erheben. Die Bilddaten können dabei insbesondere in Abhängigkeit von den CAD/CAM Daten der zu bearbeitenden/zu fertigenden 3D-Polymerstruktur erhoben werden.

Die Bilddaten werden, gemäß den Ansprüchen, mit den CAD-Daten verglichen und bei Feststellen einer Abweichung des bereits (teil-)gefertigten 3D-Polymerstruktur die CAD/CAM-Daten für das Erzeugen der übrigen 3D-Polymerstruktur auf Grundlage der Bilddaten geändert. Dadurch kann die 3D-Polymerstruktur mit außergewöhnlich kleiner Toleranz bearbeitet/gefertigt werden.

Bei der 3D-Polymerstruktur kann es sich erfindungsgemäß um ein Traggerüst für Gewebe, ein Organ, insbesondere einen Knochen, eine Niere, eine Leber, einen Knorpelersatz, einen Knochenersatz oder einen Gefäßabschnitt, handeln.

Weitere Vorteile der Erfindung ergeben sich aus der Beschreibung und der Zeichnung. Die gezeigten und beschriebenen Ausführungsformen sind nicht als abschließende Aufzählung zu verstehen, sondern haben vielmehr beispielhaften Charakter für die Schilderung der Erfindung.

### Detaillierte Beschreibung der Erfindung und Zeichnung

- Fig. 1: zeigt den schematischen Aufbau einer erfindungsgemäßen Vorrichtung und mit einer im Arbeitsbereich der Vorrichtung anordenbaren Polymervorstufe bzw. 3D-Polymerstruktur;
- Fig. 2: zeigt die 3D-Polymerstruktur oder Polymervorstufe mit der paramagnetischen Substanz in einem ausgewählten Voxel;
- Fig. 3: zeigt eine Umhausung, innerhalb derer die Polymerstruktur Polymervorstufe während des 3D-Bearbeitungs- oder Fertigungsprozesses angeordnet werden kann; und
- Fig. 4: zeigt ein Blockschaltbild des erfindungsgemäßen Verfahrens zum Bearbeiten bzw. Erzeugen einer 3D-Polymerstruktur mit dessen einzelnen Verfahrensschritten.

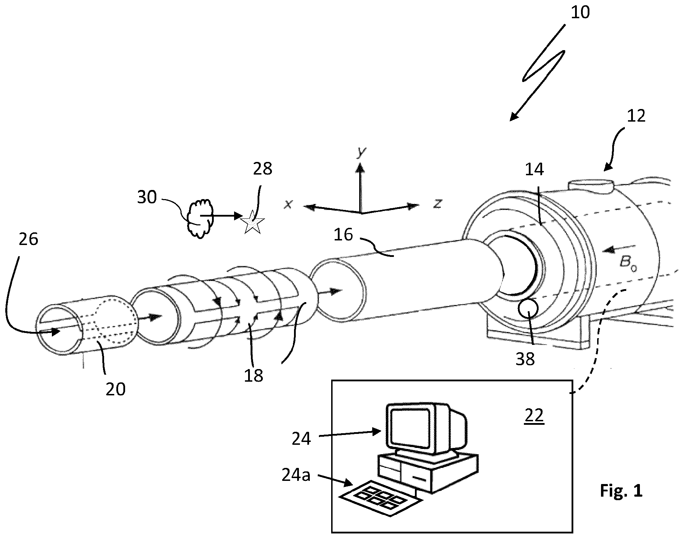

**Fig. 1** zeigt eine Vorrichtung **10** mit einem Gehäuse **12,** die einen Magnetfeldgenerator **14** zum Erzeugen eines statischen Magnetfeldes B₀, optionale Shimspulen **16** und Gradientenspulen **18,** ein HF-Feldgenerator **20,** eine Steuerungseinheit **22** mit einem Computersystem **24** und einer Eingabe- und Bedienkonsole **24a,** sowie einen im Gehäuse 12 angeordneten Arbeitsbereich **26** aufweist. In dem Arbeitsbereich 26 ist eine zu bearbeitende (zumindest teilpolymerisierte oder auspolymerisierte) 3D-Polymerstruktur **28** bzw. eine Polymervorstufe **30** (= ein Präpolymer) anordenbar, die als Ausgangsmaterial für die mit der Vorrichtung 10 zu erzeugende 3D-Polymerstruktur dient.

Der Magnetfeldgenerator 14 dient dazu, im Arbeitsbereich 26 ein homogenes statisches Magnetfeld B₀ (nachfolgend kurz: B₀-Feld) zu erzeugen. Die Feldstärke des B₀-Felds ist dabei um Potenzen größer, als das Erdmagnetfeld. Der Magnetfeldgenerator 14 kann beispielsweise einen Permanentmagneten oder einen supraleitenden Magneten umfassen. Dieses homogene B₀-Feld kann durch die Gradientenspulen 18 gezielt verändert werden. Die Gradientenspulen 18 befinden sich vorzugsweise am Umfang des Arbeitsbereichs 26. Mit diesen Gradientenspulen 18 können dem statischen B₀-Feld kontinuierlich ansteigende oder abfallende Magnetfelder, sogenannte Gradientenfelder, in mindestens allen drei Raumrichtungen x, y, z, in definierter Weise überlagert werden.

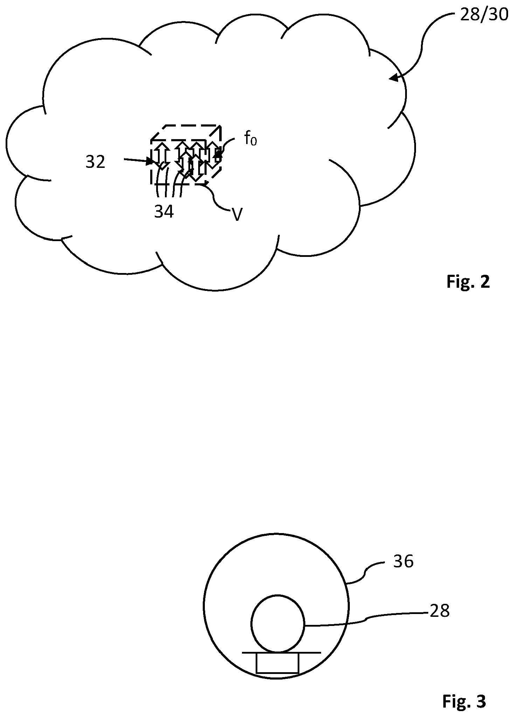

Die Polymerstruktur 28/Polymervorstufe 30 umfasst gemäß der schematischen Darstellung eines Voxels **V** der 3D-Polymerstruktur 28/Polymervorstufe 30 in **Fig. 2** eine paramagnetische Substanz **32,** die in der 3D-Polymerstruktur 28/ Polymervorstufe 30 vorzugsweise homogen verteilt angeordnet ist. Die paramagnetische Substanz 32 ist vorzugsweise durch superparamagnetische nanopartikuläre Metallpartikel **34** (=Nano-Oszillatoren), gebildet. Die Metallpartikel 34 können insbesondere aus nanopartikulärem Magnetit (Fe₃O₄) oder einem Silberhalogenid (AgₙXₙ) bestehen. Ein Kubikmillimeter (mm³) der 3D-Polymerstruktur 28 bzw. der Polymervorstufe 30 enthält vorzugsweise mehr als 1000, bevorzugt mehr als 10.000, und bis zu 10¹⁷ der Metallpartikel 34.

Im statischen B₀-Feld richten alle Metallpartikel 34 ihre Rotationsmomente parallel bzw. antiparallel zu den Magnetfeldlinien des B₀-Felds aus. Damit geht eine Synchronisierung ihrer Resonanzfrequenz-Empfindlichkeit für eingestrahlte HF-Strahlung bzw. HF-Impulse einher. Die Resonanzfrequenz-Empfindlichkeit hängt neben dem Material, der Polarität und der Geometrie und Größe der Metallpartikel 34 von der Feldstärke des B₀-Felds ab. Für jede Feldstärke des B₀-Felds existiert mithin im Wesentlichen nur eine einzige, spezifische Frequenz der elektromagnetischen HF-Strahlung, welche alle in gleicher Weise konfektionierten Metallpartikel als Oszillatoren maximal stimuliert - die sogenannte Resonanzfrequenz.

Bei einfachen (theoretischen) Systemen ohne Dämpfung ist die Resonanzfrequenz gleich der ungedämpften Eigenfrequenz (Kennfrequenz) f₀ der paramagnetischen Metallpartikel 34. Bei gedämpften Systemen ist die Frequenz, bei der die maximale Amplitude auftritt, stets kleiner als die ungedämpfte Eigenfrequenz.

Unter Resonanzbedingungen weisen also alle durch die HF-Strahlung anregbaren Metallpartikel 34 eine maximale Energieabsorptionsbereitschaft hinsichtlich des spezifischen HF-Wechselfeldes auf. Dies resultiert in einer signifikant erhöhten Oszillationsbewegung der Metallpartikel 34. Diese Oszillationsbewegungen werden (auf molekularer Ebene) aufgrund von Widerstands- und Reibungseffekten mit minimalem räumlichem Kopplungsabstand in thermische Energie umgewandelt. Diese kann zum (lokal begrenzten) Destruieren der 3D-Polymerstruktur 28 bzw. zum Polymersieren der Polymervorstufe 30 führen. Die Metallpartikel 34 können also insoweit als Nano-Oszillatoren bezeichnet werden.

Die dynamischen Gradientenfelder (B1, B2, B3; in der Zeichnung nicht dargestellt) der Vorrichtung 10 dienen in einer zur Kernspintomographie entsprechenden Weise zur Schichtselektion und Ortscodierung in mindestens 3 Raumrichtungen, d.h. funktionell zur "indirekten Fokussierung" der mittels des HF-Feldgenerators 20 in den Arbeitsbereich eingestrahlten HF-Strahlung.

Die Gradientenfelder sind durch einen kontinuierlichen Anstieg bzw. Abfall der jeweiligen Magnetfeldstärke entlang ihrer charakteristischen Achsen x, y, z, relativ zum B₀-Feld gekennzeichnet. An dieser Stelle sei auf die dem Fachmann geläufigen und gleichartigen Gradientenfelder bei der Kernspintomografie verwiesen. Ein zugeschaltetes Gradientenfeld in der x-Achse überlagert mithin das zuvor homogene permanente statische B₀-Feld und führt somit zu einem linearen Anstieg bzw. Abfall der statischen Gesamtfeldstärke entlang der x-Achse. Gleiches gilt für jeweils zugeschaltete Gradientenfelder entlang der y-Achse und z-Achse. Dies hat zur Folge, dass jedes Metallpartikel 34 oder jedes Raumvolumen bzw. Voxel der Polymervorstufe 30 im dreidimensionalen Raum über seine individuelle, ihm eigene elektromagnetische Nische (= Ortscodierung) im Schnittpunkt der mindestens 3 Gradientenfelder verfügt.

Unterscheidet sich also das magnetische Mikromilieu jedes Oszillators bzw. jedes Voxels der 3D-Polymerstruktur 28/der Polymervorstufe 30 in mindestens allen drei Raumrichtungen linear vom magnetischen Mikromilieu seines Nachbar-Oszillators bzw. benachbarten Voxels (= Nachbarvoxel), dann verfügt jedes Nanopartikel bzw. verfügen die Nanopartikel jedes einzelnen Voxels über seine/ihre ganz eigene individuelle Resonanzfrequenz und lässt/lassen sich folglich individuell isoliert adressieren und durch ein ungerichtetes elektromagnetisches HF-Feld selektiv anregen. Hierbei definiert die jeweilige Steilheit der x-, y-, z- Gradientenfelder die Kantenlängen der Voxel in allen 3 Raumrichtungen x, y, z, optimalerweise durch simultane summarische Überlagerung ihrer lokalen Feldstärke, alternativ sequentiell, z.B. unter Verwendung der inversen Fourier-Transformation.

Die Steuerungseinheit 22 der Vorrichtung 10 ist eingerichtet, den HF-Feldgenerator 20 derart anzusteuern, dass HF-Strahlung mit einer auf die Resonanzfrequenz der Metallpartikel/Oszillatoren abgestimmten Feldfrequenz in den Arbeitsbereich 26 eingestrahlt wird, um die 3D-Polymerstruktur 28 bzw. die Polymervorstufe 30 im zuvor ortscodierten Voxel V oder in der zuvor ortscodierten zusammenhängenden Volumeneinheit der 3D-Polymerstruktur 28/Polymervorstufe 30 "en bloc" lokal zu erhitzen und zu zerstören bzw. im Falle der Polymervorstufe 30 zu polymerisieren.

Die typische Feldfrequenz der HF-Strahlung zur Oszillationsanregung der paramagnetischen Substanz beträgt dabei zwischen 100 oder 108 KHz und 789 THz.

Die Steuerungseinheit 22 der Vorrichtung 10 weist, gemäß Anspruch 1, einen Betriebsmodus auf, der dem Gewinnen von Bilddaten (z.B. magnetresonanztomografischen Bilddaten) aus dem Arbeitsbereich 26 bzw. der 3D-Polymerstruktur 28 bzw. der Polymervorstufe 30 dient. Die Steuerungseinheit 22, insbesondere das Computersystem 24, dient dem Steuern aller Betriebsprozesse der Vorrichtung 10 anhand von vorgegebenen CAD/CAM-Daten. Darüber hinaus ist die Steuerungseinheit 22 gemäß Anspruch 1 dazu eingerichtet, die Bilddaten auszuwerten. So ist die Steuerungseinheit 22 dazu eingerichtet, die CAD/CAM-Daten mit den zuvor gewonnenen Bilddaten zu vergleichen und bei Feststellen einer Abweichung, die größer ist, als eine vorgegebene zulässige maximale Abweichung, den weiteren Bearbeitungs- bzw. Fertigungsprozess auf Basis der Bilddaten fortzusetzen. Hier ist die Steuerungseinheit eingerichtet, die CAD/CAM-Daten betreffend die übrige zu bearbeitende bzw. zu erzeugende 3D-Polymerstruktur zu ändern. Auf diese Weise kann die 3D-Polymerstruktur mit besonders kleinen Toleranzen bearbeitet/gefertigt werden.

Der Arbeitsbereich 26 der Vorrichtung kann gemäß Fig. 3 mittels einer, bevorzugt gasdichten, Umhausung **36** abgegrenzt sein. Die Umhausung 36 kann beispielsweise aus Kunststoff oder aus Glas oder einem anderen gegenüber HF-Feldern bzw. Magnetfeldern nicht-schirmenden Material gebildet sein. Die Umhausung kann beispielsweise durch eine Kunststofffolie gebildet sein.

Dem Arbeitsbereich der Vorrichtung 10 kann eine Pumpe **38** (Fig. 1) zugeordnet sein, mittels derer die Atmosphäre in der Umhausung 36 evakuierbar oder im Wesentlichen evakuierbar ist und/oder über die der Arbeitsbereich innerhalb der Umhausung 36 mit einem für den Fertigungsprozess vorgegebenen Fluid, insbesondere einer Arbeitsatmosphäre A, befüllbar ist. Auf diese Weise kann beispielsweise unerwünschten oxidativen Prozessen der 3D-Polymerstruktur 28/Polymervorstufe 30 durch in der Arbeitsatmosphäre A enthaltenen Sauerstoff entgegengewirkt werden.

Die Vorrichtung 10 kann insbesondere durch ein modifiziertes MRT-Gerät gebildet sein, dessen Steuerungseinheit 22 in der vorstehend erläuterten Weise an die Bearbeitung bzw. Fertigung der 3D-Polymerstruktur adaptiert ist.

Die Polymervorstufe 30 kann gleiche oder unterschiedliche Monomere bzw. Polymere (insbesondere auch Dimere, Oligomere) aufweisen. Darüber hinaus kann die 3D-Polymerstruktur 28/Polymervorstufe 30 Fasern und/oder einen oder mehrere andere Zuschlagstoffe, wie diese eingangs erläutert sind, umfassen. Rein beispielhaft seien hier Zuschlagstoffe aus der Gruppe der Farbstoffe, der antibakteriellen Substanzen, der Antibiotika oder der Wachstumsfaktoren, umfassen.

In Abhängigkeit von den an die 3D-Polymerstruktur gestellten mechanischen, elektrischen oder biologischen Anforderungen kann die Polymervorstufe 30 eine Viskosität von ungefähr 10² mPa·s bis 10⁵ mPa·s oder größer aufweisen. Soll die 3D-Polymerstruktur 28 beispielsweise als Implantat am Menschen/Tier eingesetzt werden, so ist die Polymervorstufe 30 im nicht-auspolymerisierten Zustand vorzugsweise für den tierischen/menschlichen Organismus nicht toxisch und bevorzugt durch körpereigene Enzyme abbaubar bzw. per viam naturalis aus dem menschlichen/tierischen Körper eliminierbar.

Die Vorrichtung 10 kann universell eingesetzt werden. So können damit beispielsweise medizinische Implantate, insbesondere Knochenersatz, Traggerüste für Gewebe/Organe oder Gefäßprothesen erzeugt werden.

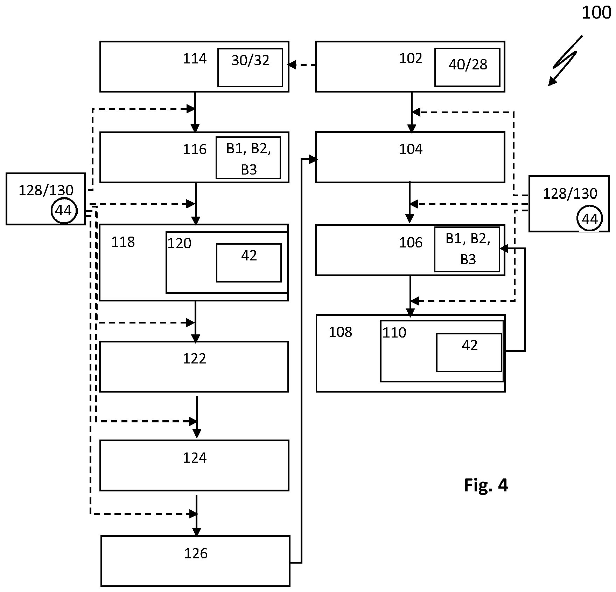

Nachstehend ist das Verfahren **100** zum Bearbeiten bzw. Erzeugen einer 3D-Polymerstruktur mit einer im Material der 3D-Polymerstruktur möglichst homogen verteilt angeordneten paramagnetischen Substanz 32 (vgl. Fig. 2) unter zusätzlicher Bezugnahme auf das in **Fig. 4** gezeigte Blockschaubild näher erläutert.

Das Verfahren 100 setzt zwingend den Einsatz des vorstehend im Kontext mit den Fign. 1 bis 3 erläuterten Vorrichtung 10 voraus und umfasst die folgenden Schritte:
a) Definieren **102** von CAD/CAM-Daten **40** der 3D-Polymerstruktur 28;
b) Bereitstellen **104** der 3D-Polymerstruktur 28 im Arbeitsbereich 26 der Vorrichtung 10;
c) Ortskodieren **106** eines Voxels V innerhalb der 3D-Polymerstruktur 28 in Abhängigkeit von den CAD/CAM Daten 40 durch Anlegen magnetischer Gradientenfelder B₁, B₂, B₃;
d) Zerstören **108** der 3D-Polymerstruktur 28 in dem zumindest einen ortskodierten Voxel V durch Einstrahlen **110** von HF-Strahlung **42** mittels derer die paramagnetische Substanz 32 in dem jeweiligen Voxel V zu destruktiven thermogenen Oszillationen angeregt wird; und
e) Wiederholen der Schritte 106 und 108 für weitere Voxel V innerhalb der 3D-Polymerstruktur 28, d. h. sequentielles Ortskodieren weiterer, bevorzugt räumlich jeweils aneinander angrenzender, Voxel V in der 3D-Polymerstruktur 28 in Abhängigkeit von den CAD/CAM-Daten und Zerstören der 3D-Polymerstruktur 28 im jeweilig weiteren ortscodierten Voxels V durch Einstrahlen (110) von HF-Strahlung 42, mittels derer die paramagnetische Substanz (32) in dem jeweiligen weiteren Voxel V zu destruktiven thermogenen Oszillationen angeregt wird, bis die 3D-Polymerstruktur (28) bearbeitet ist.

Zum Erzeugen der 3D-Struktur 28 innerhalb des Arbeitsbereichs 26 der Vorrichtung 10 aus einer Polymervorstufe dienen nach dem Schritt 102 des Definierens der CAD/CAM Daten der Polymerstruktur die folgenden Verfahrensschritte:
- Anordnen **114** einer Polymervorstufe 30 mit einer darin homogen oder im Wesentlichen homogen verteilten paramagnetischen Substanz 32 im Arbeitsbereich 26 der Vorrichtung 10;
- Ortskodieren **116** eines Voxels V innerhalb der Polymervorstufe 30 in Abhängigkeit von den CAD/CAM-Daten durch Anlegen magnetischer Gradientenfelder B₁, B₂, B₃;
- Polymerisieren **118** der Polymervorstufe 30 in dem zumindest einen ortskodierten Voxel V durch Einstrahlen **120** von HF-Strahlung **42,** mittels derer die paramagnetische Substanz 32 in dem jeweiligen weiteren Voxel V zu thermogenen Oszillationen angeregt wird; und
- nachfolgend sequentielles Ortskodieren **122** weiterer, bevorzugt räumlich jeweils aneinander angrenzender, Voxel V in der Polymervorstufe 30 in Abhängigkeit von den CAD/CAM-Daten 40 und Polymerisieren **124** der Polymervorstufe in den jeweilig weiteren ortscodierten Voxeln V durch Einstrahlen **126** von HF-Strahlung 42, mittels derer die paramagnetische Substanz 32 in dem jeweiligen weiteren Voxel V zu thermogenen Oszillationen angeregt wird, bis die zu fertigende 3D-Polymerstruktur polymerisiert ist.

Die Frequenz f der HF-Strahlung 42, d. h. des angelegten HF-Felds, ist vorzugsweise auf die jeweilige Resonanzfrequenz f₀ der mit der HF-Strahlung anzuregenden paramagnetischen Substanz 32 bzw. die Metallpartikel 34 der Polymervorstufe 30 abgestimmt bzw. stimmt mit dieser überein. Die Resonanzfrequenz f₀ der jeweiligen paramagnetischen Substanz 32 kann experimentell bestimmt werden.

Zu beachten ist, dass die Voxel V der 3D-Polymerstruktur bzw. der Polymervorstufe jeweils eine einheitliche Größe aufweisen können oder sich in ihrer Größe zumindest teilweise voneinander unterscheiden können.

Nach einer Weiterbildung der Erfindung können in einem optionalen Schritt oder mehreren optionalen Schritten **128,** insbesondere magnetresonanztomografisch oder im Wege eines alternativen Bildgebungsverfahrens, Bilddaten **44** der nichtpolymerisierten und/oder der polymerisierten Polymervorstufe 30, gewonnen werden. Schritte 124 können nach jedem anderen Verfahrensschritt erfolgen.

Die Bilddaten 44 werden dabei in einem Schritt **130** mit den CAD/CAM Daten 40 verglichen und die CAD/CAM-Daten 40 für die 3D-Polymerstruktur 28 bei Überschreiten einer maximalen Abweichung des bereits erzeugten 3D-Polymerstruktur 28 auf Grundlage der Bilddaten für das Erzeugen der übrigen 3D-Polymerstruktur 28 geändert werden. Auf diese Weise kann die 3D-Polymerstruktur 28 mit einer besonders geringen Toleranz bearbeitet bzw. erzeugt werden.

Mittels des erfindungsgemäßen Verfahrens 100 können wie eingangs beschrieben, beliebige 3D-Polymerstrukturen, beispielsweise Gebrauchsgegenstände, Maschinenteile, Traggerüste für Zellen, Gewebe, Organe bearbeitet und fakultativ auch erzeugt werden.

## Patentansprüche

1. Vorrichtung (10) zum Bearbeiten einer 3D-Polymerstruktur (28) mit einer im Material der 3D-Polymerstruktur (28) möglichst homogen verteilt angeordneten paramagnetischen Substanz (32),
umfassend:
• einen Magnetfeldgenerator (14) zum Erzeugen eines statischen Magnetfeldes B₀ in einem Arbeitsbereich (26) der Vorrichtung (10), in dem die 3D-Polymerstruktur (28) anordenbar ist;
• Gradientenspulen (18) zum Erzeugen von magnetischen Gradientenfeldern in mindestens allen drei Raumrichtungen x, y, z, mittels derer die paramagnetische Substanz (32) in einem definierten Voxel (V) der 3D-Polymerstruktur (28) ortscodierbar ist;
• einen Hochfrequenz- (HF-) Feldgenerator (20) zum Einstrahlen von HF-Strahlung (42) in den Arbeitsbereich (26); und
• eine Steuerungseinheit (22), die dazu eingerichtet ist, den HF-Feldgenerator (20) derart anzusteuern, dass die ortskodierte paramagnetische Substanz (32) im dem Voxel (V) mittels einer auf die paramagnetische Substanz (32) abgestimmten Feldfrequenz der HF-Strahlung (42) anregbar ist, um die 3D-Polymerstruktur (28) alleinig in dem definierten Voxel (V) zu zerstören,
wobei die Vorrichtung (10) ein MRT-Gerät oder eine andere Bildgebungseinheit zum Gewinnen von Bilddaten (44) umfasst,
**dadurch gekennzeichnet,**
**dass** die Steuerungseinrichtung (22) dazu eingerichtet ist, Bilddaten (44) der 3D-Polymerstruktur (28) mit CAD/CAM Daten (40) für die 3D-Polymerstruktur (28) zu vergleichen und bei Feststellen von, insbesondere geometrischen, Abweichungen der teilbearbeiteten/teilerzeugten 3D-Polymerstruktur (28) von den CAD/CAM-Daten (40) die Bilddaten (44) bzw. die Abweichungen beim weiteren Bearbeiten/Erzeugen der 3D-Polymerstruktur (28) zu berücksichtigen.

2. Vorrichtung (10) gemäß Anspruch 1, **dadurch gekennzeichnet, dass** die Vorrichtung (10) auch zum Erzeugen der 3D-Polymerstruktur (28) aus einer Polymervorstufe (30) mit einer darin homogen oder im Wesentlichen homogen verteilt angeordneten paramagnetischen Substanz (32) eingerichtet ist, wobei die Polymervorstufe (30) im Arbeitsbereich (26) der Vorrichtung (10) anordenbar ist, wobei mittels der Gradientenspulen (18) magnetische Gradientenfelder in mindestens allen drei Raumrichtungen x, y, z generierbar sind, um die paramagnetische Substanz (32) zeitlich sequenziell in definierten Voxeln V der Polymervorstufe (30) ortszucodieren und die Steuerungseinheit (22) dazu eingerichtet ist, den HF-Feldgenerator (20) derart anzusteuern, dass die paramagnetische Substanz (32) im dem jeweilig ortscodierten Voxel (V) mittels einer auf die paramagnetische Substanz (32) abgestimmten Feldfrequenz f der HF-Strahlung (42) derart anregbar ist, dass eine thermische Polymerisation der Polymervorstufe (30), bevorzugt alleinig, in dem definierten Voxel (V) ermöglicht ist.

3. Vorrichtung (10) gemäß Anspruch 1 oder 2, **dadurch gekennzeichnet, dass** die Feldfrequenz f der HF-Strahlung (42) zwischen 100 KHz oder 108 KHz und 789 THz beträgt.

4. Vorrichtung (10) gemäß einem der vorhergehenden Ansprüche, **dadurch gekennzeichnet, dass** der Arbeitsbereich (26) innerhalb einer Umhausung (36) angeordnet ist.

5. Verfahren (100) zum Bearbeiten einer 3D-Polymerstruktur (28) mit einer im Material der 3D-Polymerstruktur (28) möglichst homogen verteilt angeordneten paramagnetischen Substanz (32) mittels einer Vorrichtung (10) gemäß einem der Ansprüche 1 bis 4, umfassend die folgenden Schritte:
a. Definieren (102) von CAD/CAM-Daten der 3D-Polymerstruktur;
b. Anordnen (104) der 3D-Polymerstruktur (28) im Arbeitsbereich (26) der Vorrichtung (10);
c. Ortskodieren (106) eines Voxels (V) innerhalb der 3D-Polymerstruktur (28) in Abhängigkeit von den CAD/CAM Daten durch Anlegen magnetischer Gradientenfelder;
d. Zerstören (108) der 3D-Polymerstruktur (28) alleinig in dem zumindest einen ortskodierten Voxel (V) durch Einstrahlen (110) von HF-Strahlung (42) mittels derer die paramagnetische Substanz (32) in dem jeweiligen Voxel (V) zu destruktiven thermogenen Oszillationen angeregt wird;
und
e. Wiederholen der Schritte c) und d) für weitere Voxel (V) innerhalb der 3D-Polymerstruktur (28), d. h. sequentielles
▪ Ortskodieren (106) weiterer, bevorzugt räumlich jeweils aneinander angrenzender, Voxel (V) in der 3D-Polymerstruktur (28) in Abhängigkeit von den CAD/CAM-Daten und
▪ Zerstören (108) der 3D-Polymerstruktur (28) des jeweilig weiteren ortscodierten Voxels (V) durch Einstrahlen (110) von HF-Strahlung (42), mittels derer die paramagnetische Substanz (32) in dem jeweiligen weiteren Voxel (V) zu destruktiven thermogenen Oszillationen angeregt wird,
wobei das Bearbeiten der 3D-Polymerstruktur nicht in-vivo erfolgt.

6. Verfahren (100) gemäß Anspruch 5, **dadurch gekennzeichnet, dass** die 3D-Polymerstruktur mittels der Vorrichtung (10) erzeugt wird.

7. Verfahren (100) gemäß Anspruch 5 oder 6, **gekennzeichnet durch** die weiteren Schritte:
f. Anordnen (114) einer Polymervorstufe (30) mit einer darin homogen oder im Wesentlichen homogen verteilten paramagnetischen Substanz (32);
g. Ortskodieren (116) zumindest eines Voxels (V) innerhalb der 3D-Polymervorstufe (30) in Abhängigkeit von den CAD/CAM Daten (40) durch Anlegen magnetischer Gradientenfelder; und
h. Polymerisieren (118) der Polymervorstufe (30) in dem zumindest einen ortskodierten ersten Voxel (V) durch Einstrahlen (120) von HF-Strahlung (42) mittels derer die paramagnetische Substanz (32) in dem jeweiligen weiteren Voxel (V) zu thermogenen Oszillationen angeregt wird; und
i. Wiederholen der Schritte g) und h) zum Erzeugen der 3D-Polymerstruktur.

8. Verfahren (100) gemäß einem der vorhergehenden Ansprüche 5 bis 7, **dadurch gekennzeichnet, dass** die Frequenz f der HF-Strahlung (42), d. h. des angelegten HF-Feldes, in Abhängigkeit von der Resonanzfrequenz f₀ der mit der HF-Strahlung (42) anzuregenden paramagnetischen Substanz (32) gewählt wird.

9. Verfahren (100) gemäß einem der vorhergehenden Ansprüche 5 bis 8, **dadurch gekennzeichnet, dass** die Voxel (V) jeweils mit einer einheitlichen Größe definiert werden oder dass die Voxel (V) zumindest teilweise mit einer unterschiedlichen Größe definiert werden.

10. Verfahren (100) gemäß einem der Ansprüche 5 bis 9, **dadurch gekennzeichnet, dass** zur 3D-Polymerstruktur, insbesondere magnetresonanztomografisch, Daten (44), gewonnen werden und die weitere Bearbeitung der 3D-Polymerstruktur bzw. das weitere Erzeugen der 3D-Polymerstruktur unter Berücksichtigung dieser magnetresonanztomografischen Daten erfolgt.

11. Verfahren (100) gemäß einem der Ansprüche 5 bis 10, **dadurch gekennzeichnet, dass** als paramagnetische Substanz (32) Metallpartikel (34) in Form von nanopartikulären Magnetitpartikeln oder nanopartikulären Eisenpartikeln oder Metallorganyle verwendet wird.

12. Verfahren (100) gemäß Anspruch 11, **dadurch gekennzeichnet, dass** die Metallpartikel (34) in einer Konzentration von > 1000 Partikel pro Kubikmillimeter der Polymervorstufe, insbesondere in einer Konzentration von > 10.000 Partikel pro Kubikmillimeter der 3D-Polymerstruktur und/oder der Polymervorstufe, enthalten sind.

13. Verfahren (100) gemäß einem der vorhergehenden Ansprüche 5 bis 12, **dadurch gekennzeichnet, dass** das Material der 3D-Polymerstruktur (28) oder der Polymervorstufe (30) einen oder mehrere Zuschlagstoffe, insbesondere aus der Gruppe der Fasern, Farbstoffe, antibakteriellen Substanzen, Wachstumsfaktoren, Nanopartikel/-tubes, mineralischen Füllstoffe, metallischen Werkstoffe, Glykosaminoglykane, MMC-Stoffe, Polypeptid-Motive, Promotoren, Terminatoren, Inhibitoren, Katalysatoren, Sensitizer und/oder Immunmodulatoren umfasst.

## Claims

1. A device (10) for processing a 3D polymer structure (28) with a paramagnetic substance (32) distributed as homogeneously as possible in the material of the 3D polymer structure (28),
comprising:
• a magnetic field generator (14) for generating a static magnetic field B₀ in a working zone (26) of the device (10), in which the 3D polymer structure (28) can be arranged;
• gradient coils (18) for generating magnetic gradient fields in at least all three spatial directions x, y, z, by means of which the paramagnetic substance (32) can be spatially encoded in a defined voxel (V) of the 3D polymer structure (28);
• a radio frequency (RF) field generator (20) for irradiating RF radiation (42) into the working zone (26); and
• a control unit (22) which is configured to control the RF field generator (20) in such a way that the spatially encoded paramagnetic substance (32) in the voxel (V) can be excited by means of a field frequency of the RF radiation (42) tuned to the paramagnetic substance (32) in order to destroy the 3D polymer structure (28) solely in the defined voxel (V),
whereby the device (10) comprises an MRI unit or a different imaging appliance for obtaining image data (44), **characterized in that** the control unit (22) is configured to compare image data (44) of the 3D polymer structure (28) with CAD/CAM data (40) for the 3D polymer structure (28) and, if deviations of the partially processed/partially generated 3D polymer structure (28) from the CAD/CAM data (40) are detected, in particular geometric deviations, to take into account the image data (44) and/or the deviations during the further processing/generation of the 3D polymer structure (28).

2. The device (10) according to claim 1 **characterized in that** the device (10) is also configured to generate the 3D polymer structure (28) from a polymer precursor (30) with a paramagnetic substance (32) distributed homogeneously or essentially homogeneously distributed therein, whereby the polymer precursor (30) can be arranged in the working zone (26) of the device (10), wherein magnetic gradient fields can be generated in at least all three spatial directions x, y, z by means of the gradient coils (18) in order to spatially encode the paramagnetic substance (32) sequentially in time in defined voxels (V) of the polymer precursor (30), and
the control unit (22) is configured to control the RF field generator (20) in such a way that the paramagnetic substance (32) in the respective spatially encoded voxel (V) can be excited by means of a field frequency f of the RF radiation (42) tuned to the paramagnetic substance (32) in such a way that thermal polymerization of the polymer precursor (30) is ensured, preferably solely, in the defined voxel (V).

3. The device (10) according to claim 1 or 2, **characterized in that** the field frequency f of the RF radiation (42) is between 100 KHz and 108 KHz and 789 THz.

4. The device (10) according to any of the preceding claims, **characterized in that** the working zone (26) is arranged within a housing (36).

5. Method (100) for processing a 3D polymer structure (28) with a paramagnetic substance (32) distributed as homogeneously as possible in the material of the 3D polymer structure (28) by means of an device (10) according to any of the claims 1 to 4, comprising the following steps:
a. defining (102) CAD/CAM data for the 3D polymer structure;
b. arranging (104) the 3D polymer structure (28) in the working zone (26) of the device (10);
c. spatially encoding (106) a voxel (V) within the 3D polymer structure (28) as a function of the CAD/CAM data by applying magnetic gradient fields;
d. destroying (108) the 3D polymer structure (28) solely in the at least one spatially encoded voxel (V) by irradiating (110) RF radiation (42) by means of which the paramagnetic substance (32) in the respective voxel (V) is excited to destructive thermogenic oscillations;
and
e. repeating the steps c) and d) for further voxels (V) within the 3D polymer structure (28), i.e. sequential
▪ spatially encoding (106) further, preferably spatially adjacent, voxels (V) in the 3D polymer structure (28) as a function of the CAD/CAM data and
▪ destroying (108) the 3D polymer structure (28) of the respective further spatially encoded voxel (V) by irradiating (110) RF radiation (42), by means of which the paramagnetic substance (32) in the respective further voxel (V) is excited to destructive thermogenic oscillations,
whereat the processing of the 3D polymer structure is not carried out in-vivo.

6. Method (100) according to claim 5, **characterized in that** the 3D polymer structure is generated by means of the device (10).

7. Method (100) according to claim 5 or 6, **characterized by** the further steps:
f. arranging (114) a polymer precursor (30) with a paramagnetic substance (32) homogeneously or substantially homogeneously distributed therein;
g. spatially encoding (116) at least one voxel (V) within the 3D polymer precursor (30) as a function of the CAD/CAM data (40) by applying magnetic gradient fields; and
h. polymerizing (118) the polymer precursor (30) in the at least one spatially encoded first voxel (V) by irradiating (120) RF radiation (42) by means of which the paramagnetic substance (32) in the respective further voxel (V) is excited to thermogenic oscillations; and
i. repeating the steps g) and h) to generate the 3D polymer structure.

8. Method (100) according to any of the preceding claims 5 to 7, **characterized in that** the frequency f of the RF radiation (42), i.e. of the applied RF field, is selected depending on the resonant frequency f₀ of the paramagnetic substance (32) to be excited with the RF radiation (42).

9. Method (100) according to any of the preceding claims 5 to 8, **characterized in that** the voxels (V) are each defined with a uniform size or **in that** the voxels (V) are at least partially defined with a different size.

10. Method (100) according to any of the claims 5 to 9, **characterized in that** data (44) are obtained for the 3D polymer structure, in particular by magnetic resonance tomography, and the further processing of the 3D polymer structure or the further manufacturing of the 3D polymer structure takes place taking into account these magnetic resonance tomography data.

11. Mathod (100) according to any of the claims 5 to 10, **characterized in that** metal particles (34) in the form of nanoparticulate magnetite particles or nanoparticulate iron particles or metal organyls are used as the paramagnetic substance (32).

12. Mathod (100) according to claim 11, **characterized in that** the metal particles (34) are present in a concentration of > 1000 particles per cubic millimetre of the polymer precursor, in particular in a concentration of > 10,000 particles per cubic millimetre of the 3D polymer structure and/or the polymer precursor.

13. Method (100) according to any of the preceding claims 5 to 12, **characterized in that** the material of the 3D polymer structure (28) or of the polymer precursor (30) comprises one or more additives, in particular from the group of fibres, dyes, antibacterial substances, growth factors, nanoparticles/tubes, mineral fillers, metallic materials, glycosaminoglycans, MMC substances, polypeptide motifs, promoters, terminators, inhibitors, catalysts, sensitizers and/or immunomodulators.

## Revendications

1. Dispositif (10) pour traitement d'une structure polymère 3D (28) avec une substance paramagnétique (32) répartie de manière aussi homogène que possible dans le matériau de la structure polymère 3D (28),
comprenant
• un générateur de champ magnétique (14) pour la génération d'un champ magnétique statique B0 dans une zone de travail (26) du dispositif (10), dans laquelle la structure polymère 3D (28) peut être disposée;
• des bobines de gradient (18) pour la génération des champs de gradient magnétique dans au moins les trois directions spatiales x, y, z, au moyen desquelles la substance paramagnétique (32) peut être codée spatialement dans un voxel (V) défini de la structure polymère 3D (28);
• un générateur de champ haute fréquence (HF) (20) pour l'irradiation d'un rayonnement HF (42) dans la zone de travail (26) ; et
• une unité de commande (22), qui est configurée pour commander le générateur de champ HF (20) de telle sorte que la substance paramagnétique (32) codée spatialement dans le voxel (V) puisse être excitée au moyen d'une fréquence de champ du rayonnement HF (42) adaptée à la substance paramagnétique (32) afin de détruire la structure polymère 3D (28) uniquement dans le voxel défini (V),
le dispositif (10) comprenant un appareil d'IRM ou une autre unité d'imagerie pour obtenir des données d'image (44), **caractérisé en ce que** le dispositif de commande (22) est configurée pour comparer les données d'image (44) de la structure polymère 3D (28) avec les données CAD/CAM (40) pour la structure polymère 3D (28) et en cas de constation des différences, en particulier des écarts géométriques, de la structure polymère 3D partiellement traitée/partiellement générée (28) par rapport aux données CAD/CAM (40), pour prendre en compte les données d'image (44) et/ou les écarts lors de la poursuite du traitement/de la génération de la structure polymère 3D (28).

2. Dispositif (10) selon la revendication 1 **caractérisé en ce que** le dispositif (10) est également configuré à générer une structure polymère 3D (28) à partir d'un précurseur polymère (30) avec une substance paramagnétique (32) répartie de manière homogène ou essentiellement homogène,
le précurseur de polymère (30) étant placé dans la zone de travail (26) du dispositif (10), les bobines de gradient (18) permettant de générer des champs magnétiques de gradient dans au moins trois directions spatiales x, y, z, afin que la substance paramagnétique (32) puisse être codée sequentiellement dans des voxels (V) définis du précurseur de polymère (30)
et
l'unité de commande (22) est configurée pour commander le générateur de champ HF (20) de telle sorte que la substance paramagnétique (32) dans le voxel (V) respectif codé spatialement puisse être excitée au moyen d'une fréquence de champ f du rayonnement HF (42) adaptée à la substance paramagnétique (32) de manière à assurer la polymérisation thermique du précurseur de polymère (30), de préférence uniquement, dans le voxel (V) défini.

3. Dispositif (10) selon la revendication 1 ou 2, **caractérisé en ce que** la fréquence de champ f du rayonnement HF (42) se situe entre 100 KHz et 108 KHz et 789 THz.

4. Dispositif (10) selon l'une des revendications précédentes, **caractérisé en ce que** la zone de travail (26) est disposée à l'intérieur d'une inceinte (36).

5. Procédé (100) pour le traitment d'une structure polymère 3D (28) avec une substance paramagnétique (32) répartie de manière aussi homogène que possible dans le matériau de la structure polymère 3D (28) au moyen d'un dispositif (10) selon l'une des revendications 1 à 4, comprenant les étapes suivantes :
a. définition (102) de données CAD/CAM pour la structure polymère 3D ;
b. disposer (104) la structure polymère 3D (28) dans la zone de travail (26) du dispositif (10) ;
c. codage spatial (106) d'un voxel (V) à l'interiereur de la structure polymère 3D (28) en fonction des données CAD/CAM par application de champs de gradient magnétiques ;
d. déstruction (108) de la structure polymère 3D (28) uniquement dans l'au moins un voxel (V) codé spatialement par irradiation (110) de rayonnement HF (42) au moyen duquel la substance paramagnétique (32) dans le voxel (V) respectif est excitée en oscillations thermogéniques destructrices ;
et
e. répétition des étapes c) et d) pour d'autres voxels (V) à l'intérieur de la structure polymère 3D (28), c'est-à-dire séquentiellement:
- codage spatiale (106) d'autres voxels (V), de préférence spatialement adjacents, dans la structure polymère 3D (28) en fonction des données CAD/CAM et
- déstruction (108) de la structure polymère 3D (28) de l'autre voxel (V) respectif codé spatialement par irradiation (110) d'un rayonnement HF (42), au moyen duquel la substance paramagnétique (32) dans cet autre voxel respectif (V) est excitée en oscillations thermogéniques destructrices, le traitement de la structure polymère 3D n'étant pas effectué in vivo.

6. Procédé (100) selon la revendication 5, **caractérisé par le fait que** la structure polymère 3D est générée au moyen du dispositif (10).

7. Procédé (100) selon la revendication 5 ou 6, **caractérisé par** les étapes suivantes :
f. disposer (114) un précurseur de polymère (30) avec une substance paramagnétique (32) distribuée de manière homogène ou substantiellement homogène ;
g. codage spatial (116) d'au moins un voxel (V) à l'intérieur du précurseur de polymère 3D (30) en fonction des données CAD/CAM (40) en appliquant des champs de gradient magnétique ; et
h. polymérisation (118) du précurseur polymère (30) dans ledit au moins un premier voxel (V) codé spatialement par irradiation (120) un rayonnement HF (42) au moyen duquel la substance paramagnétique (32) dans ledit l'autre voxel (V) respectif est excitée en oscillations thermogéniques ; et
i. répétition des étapes g) et h) pour générer la structure polymère 3D.

8. Procédé (100) selon l'une des revendications précédentes 5 à 7, **caractérisé en ce que** la fréquence f du rayonnement HF (42), c'est-à-dire du champ HF appliqué, est sélectionnée en fonction de la fréquence de résonance f₀ de la substance paramagnétique (32) à exciter avec le rayonnement HF (42).

9. Procédé (100) selon l'une des revendications précédentes 5 à 8, **caractérisé en ce que** les voxels (V) sont respectivement définis avec une taille uniforme ou que les voxels (V) sont au moins partiellement définis avec une taille différente.

10. Procédé (100) selon l'une des revendications 5 à 9, **caractérisé en ce que** des données (44) pour la structure polymère 3D sont obtenues en particulier par tomographie par résonance magnétique, et la suite de traitement de la structure polymérique 3D ou la suite de la production de la structure polymérique 3D s'effectue en tenant compte de ces données de tomographie par résonance magnétique.

11. Procédé (100) selon l'une des revendications 5 à 10, **caractérisé en ce que** des particules métalliques (34) sous forme de particules de magnétite nanoparticulaires ou de particules de fer nanoparticulaires ou d'organyles métalliques sont utilisées comme substance paramagnétique (32).

12. Procédé (100) selon la revendication 11, **caractérisé en ce que** les particules métalliques (34) sont contenues en une concentration de > 1000 particules par millimètre cube du précurseur de polymère, en particulier en une concentration de > 10 000 particules par millimètre cube de la structure polymère 3D et/ou du précurseur de polymère.

13. Procédé (100) selon l'une des revendications précédentes 5 à 12, **caractérisé en ce que** le matériau de la structure polymère 3D (28) ou du précurseur de polymère (30) comprend un ou plusieurs additifs, notamment du groupe des fibres, colorants, substances antibactériennes, facteurs de croissance, nanoparticules/tubes, charges minérales, matériaux métalliques, glycosaminoglycanes, substances MMC, motifs polypeptidiques, promoteurs, terminateurs, inhibiteurs, catalyseurs, sensibilisateurs et/ou immunomodulateurs.
